# EUROPEAN PATENT SPECIFICATION

(11) **EP 0 623 679 B1**
(45) Date of publication and mention of the grant of the patent: **25.06.2003**
(21) Application number: 94201816.9
(22) Date of filing: 19.05.1988
(51) Int. Cl.: C12N 15/62, C12N 15/13, C07K 14/00, C12N 1/20, A61K 39/395

(54) **Targeted multifunctional proteins**
Multifunktionelle Proteine mit vorbestimmter Zielsetzung
Protéines multifonctionneles à cible prédéterminée

(30) Priority: 21.05.1987 US 52800
(43) Date of publication of application: 09.11.1994
(62) Divisional of application: 88905298.1
(73) Proprietor: Micromet AG, 81477 München (DE)
(72) Inventor: Huston, James S., Newton, Massachusetts 02158 (US); Oppermann, Hermann, Medway, Massachusetts 02053 (US)
(74) Representative: Dehmel, Albrecht, Dr.

(56) References cited:
- WO-A-88/01649
- BIOTECHNOLOGY vol. 4, no. 12 , December 1986 , NEW YORK US pages 1041 - 1043 A.KLAUSNER '"Single-chain"antibodies become a reality'
- PROCEEDINGS OF THE NATIONAL ACADEMY OF SCIENCES OF USA. vol. 85 , August 1988 , WASHINGTON US pages 5879 - 5883 J.S.HUSTON ET AL. 'Protein engineering of antibody binding sites: Recovery of specific activity in an anti-digoxin single-chain Fv analogue produced in Escherichia coli'

## Description

This invention relates to novel compositions of matter, hereinafter called targeted multifunctional proteins, useful, for example, in specific binding assays, affinity purification, biocatalysis, drug targeting, imaging, immunological treatment of various oncogenic and infectious diseases, and in other contexts. More specifically, this invention relates to biosynthetic proteins expressed from recombinant DNA as a single polypeptide chain comprising plural regions, one of which has a structure similar to an antibody binding site, and an affinity for a preselected antigenic determinant, and another of which has a separate function, and may be biologically active, designed to bind to ions, or designed to facilitate immobilization of the protein. This invention also relates to the binding proteins per se, and methods for their construction.

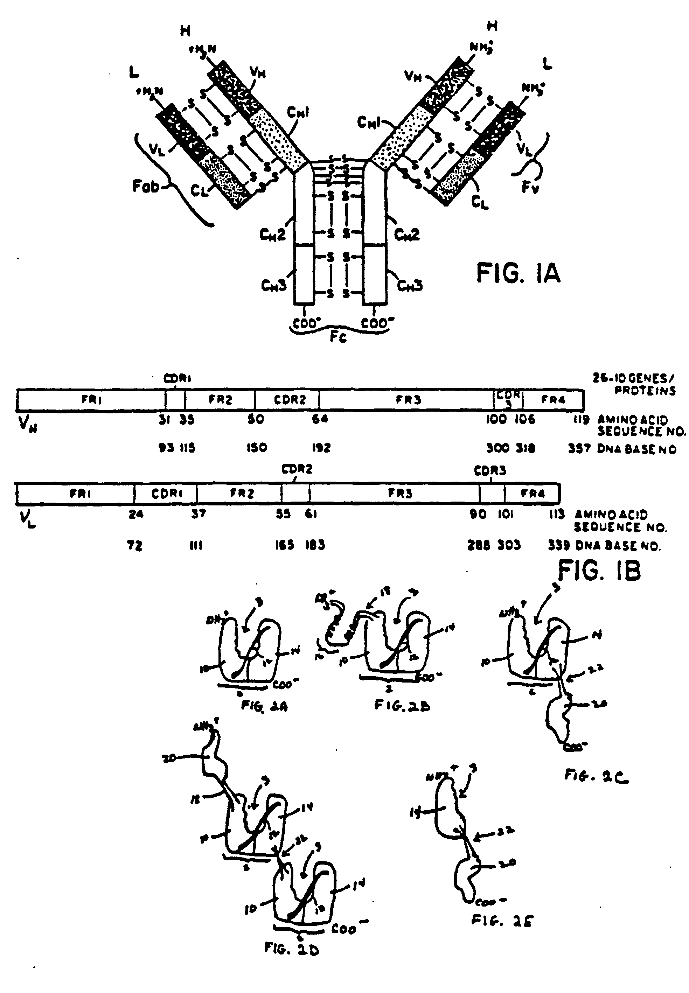

There are five classes of human antibodies. Each has the same basic structure (see Figure 1), or multiple thereof, consisting of two identical polypeptides called heavy (H) chains (molecularly weight approximately 50,000 d) and two identical light (L) chains (molecular weight approximately 25,000 d). Each of the five antibody classes has a similar set of light chains and a distinct set of heavy chains. A light chain is composed of one variable and one constant domain, while a heavy chain is composed of one variable and three or more constant domains. The combined variable domains of a paired light and heavy chain are known as the Fv region, or simply "Fv". The Fv determines the specificity of the immunoglobulin, the constant regions have other functions.

Amino acid sequence data indicate that each variable domain comprises three hypervariable regions or loops, sometimes called complementarity determining regions or "CDRs" flanked by four relatively conserved framework regions or "FRs" (Kabat et. al., Sequences of Proteins of Immunological Interest [U.S. Department of Health and Human Services, third edition, 1983, fourth edition, 1987]). The hypervariable regions have been assumed to be responsible for the binding specificity of individual antibodies and to account for the diversity of binding of antibodies as a protein class.

Monoclonal antibodies have been used both as diagnostic and therapeutic agents. They are routinely produced according to established procedures by hybridomas generated by fusion of mouse lymphoid cells with an appropriate mouse myeloma cell line.

The literature contains a host of references to the concept of targeting bioactive substances such as drugs, toxins, and enzymes to specific points in the body to destroy or locate malignant cells or to induce a localized drug or enzymatic effect. It has been proposed to achieve this effect by conjugating the bioactive substance to monoclonal antibodies (see, e.g., Vogel, Immunoconjugates. Antibody Conjugates in Radioimaging and Therapy of Cancer, 1987, N.Y., Oxford University Press; and Ghose et al. (1978) J. Natl. Cancer Inst. 61:657-676, ). However, non-human antibodies induce an immune response when injected into humans. Human monoclonal antibodies may alleviate this problem, but they are difficult to produce by cell fusion techniques since, among other problems, human hybridomas are notably unstable, and removal of immunized spleen cells from humans is not feasible.

Chimeric antibodies composed of human and non-human amino acid sequences potentially have improved therapeutic value as they presumably would elicit less circulating human antibody against the non-human immunoglobulin sequences. Accordingly, hybrid antibody molecules have been proposed which consist of amino acid sequences from different mammalian sources. The chimeric antibodies designed thus far comprise variable regions from one mammalian source, and constant regions from human or another mammalian source (Morrison et al. (1984) Proc. Natl. Acad. Sci. U.S.A., 81:5851-6855; Neuberger et al. (1984) Nature 312:604-608; Sahagan et al. (1986) J. Immunol. 137:1066-1074; EPO application nos. 04302368.0, Genentech; 85102665.3, Research Development Corporation of Japan; 85305604.2, Stanford; P.C.T. application no. PCT/GB85/00392, Celltech Limited).

It has been reported that binding function is localized to the variable domains of the antibody molecule located at the amino terminal end of both the heavy and light chains. The variable regions remain noncovalently associated (as V_{H}V_{L} dimers, termed Fv regions) even after proteolytic cleavage from the native antibody molecule, and retain much of their antigen recognition and binding capabilities (see, for example, Inbar et al., Proc. Natl. Acad. Sci. U.S.A. (1972) 69:2659-2662; Hochman et. al. (1973) Biochem. 12:1130-1135; and (1976) Biochem. 15:2706-2710; Sharon and Givol (1976) Biochem. 15:1591-1594; Rosenblatt and Haber (1978) Biochem. 17:3877-3882; Ehrlich et al. (1980) Biochem. 19:4091-40996). Methods of manufacturing two-chain Fv substantially free of constant region using recombinant DNA techniques are disclosed in U.S. 4,642,334 and corresponding published specification EP 088,994.

### Summary of the Claimed Invention

According to one aspect of the present invention there is provided a single polypeptide chain. The single polypeptide chain comprises a linking sequence which is at least 10 amino acid residues in length. The linking sequence connects a first and a second non-naturally peptide-bonded, biologically active polypeptide domain to form a single polypeptide chain. This single polypeptide chain comprises at least two biologically active domains connected by the linking sequence. The linking sequence comprises hydrophilic peptide-bonded amino acids exhibiting small and unreactive side chains but no cysteine. The hydrophilic amino acids constitute a hydrophilic sequence which has a flexible unstructured configuration essentially free of secondary structure in aqueous solution. The linking sequence contains a plurality of glycine or serine residues and spans the distance between the C-terminal end of the first domain and the N-terminal end of the second domain.

The linking sequence of the polypeptide chain described in the previous paragraph may contain threonine. The first and second non-naturally peptide-bonded, biologically active domains may be connected to the linking sequence such that the linking sequence is peptide-bonded at its N-terminus to the first biologically active domain and at its C-terminus to the second biologically active domain. The linking sequence may further comprise plural consecutive copies of an amino acid sequence, for example the amino acid sequence (GlyGlyGlyGlySer)₃ and may further comprise one or a pair of amino acid sequences recognizable by a site-specific cleavage agent.

According to another aspect of the invention there is provided a polypeptide linker. The polypeptide linker has a length of at least 10 amino acid residues and links two non-naturally linked polypeptide domains to form a multifunctional protein. The linker exhibits amino acids with small and unreactive side chains and comprises plural hydrophilic peptide-bonded amino acids constituting a hydrophilic sequence. The linker spans the distance between the C-terminal end of a first domain and the N-terminal end of a second domain. Each domain comprises a biologically active polypeptide having a conformation suitable for biological activity independent of the biological activity of the other domain.

The polypeptide linker described in the above paragraph may, independently, comprise threonine, be cysteine-free, comprise a plurality of glycine or serine residues, comprise plural consecutive copies of an amino acid sequence, span a distance of at least 4 nm (40 Angstroms), comprise the amino acid sequence (GlyGlyGlyGlySer)₃, or comprise one amino acid sequence or a pair of amino acid sequences recognizable by a site-specific cleavage agent. At least one of the polypeptide domains linked by the polypeptide linker described in the above paragraph may comprise an enzyme, a toxin, a receptor, a binding site, a biosynthetic antibody binding site, a growth factor, a cell-differentiation factor, a lymphokine, a cytokine, a hormone, a remotely detectable moiety or an anti-metabolite. The first domain linked to the polypeptide linker described in the above paragraph may comprise a single chain binding site, and the second domain linked to the polypeptide linker described in the above paragraph may comprise an enzyme, a toxin, a receptor, a binding site, a biosynthetic antibody binding site, a growth factor, a cell-differentiation factor, a lymphokine, a cytokine, a hormone or an anti-metabolite. At least one of the domains linked to the linker described in the previous paragraph may comprise a polypeptide capable of sequestering an ion, such as preferably calmodulin, methallothionein, a fragment thereof, or an amino acid sequence rich in at least one of glutamic acid, aspartic acid, lysine and arginine. The amino acids of the linker described in the previous paragraph may assume an unstructured polypeptide configuration in aqueous solution.

According to another aspect of the invention there is provided a polypeptide linker. The polypeptide linker has a length of at least 10 amino acid residues and links two non-naturally linked polypeptide domains such to form a functional protein. The linker exhibits amino acids with small and unreactive side chains and comprises plural hydrophilic peptide-bonded amino acids constituting a hydrophilic sequence. The linker spans the distance between the C-terminal end of a first domain and the N-terminal end of a second domain. Together, the domains comprise an immunologically reactive binding site for a preselected antigen.

The two polypeptide domains linked by the linker described in the previous paragraph may be of such a nature as to mimic a V_{H} and V_{L} chain from a natural immunoglobulin. The polypeptide linker described in the above paragraph may, independently, comprise threonine, be cysteine-free, comprise a plurality of glycine or serine residues, comprise plural consecutive copies of an amino acid sequence, span a distance of at least 4 nm (40 Angstroms), comprise the amino acid sequence (GlyGlyGlyGlySer)₃, or comprise one amino acid sequence or a pair of amino acid sequences recognizable by a site-specific cleavage agent. The amino acids of the linker described in the previous paragraph may assume an unstructured polypeptide configuration in aqueous solution.

Further aspects of this invention provide DNA encoding the polypeptide chain described above, DNA encoding the respective polypeptide linkers described above and a host cell transformed with and capable of expressing the DNA encoding the respective polypeptide linkers described above.

Note: The instant application is a divisional application of EP 88905298.1, now granted as EP 0 318 554. As much of the description of the parent application is necessary to understand the subject matter claimed in the instant application in its proper context, large portions of the parent description were left intact in adapting the instant description to the allowed claims. It should not, however, be inferred that subject matter contained in the instant description and covered by claims of EP 0 318 554 is included in the subject matter instantly claimed.

As used herein, the phrase biosynthetic antibody binding site or BABS means synthetic proteins expressed from DNA derived by recombinant techniques. BABS comprise biosynthetically produced sequences of amino acids defining polypeptides designed to bind with a preselected antigenic material. The definition of BABs according to the instant divisional application does not include the polypeptide constructs as claimed herein. The structure of these synthetic polypeptides is unlike that of naturally occurring antibodies, fragments thereof, e.g., Fv, or known synthetic polypeptides or "chimeric antibodies" in that the regions of the BABS responsible for specificity and affinity of binding, (analogous to native antibody variable regions) are linked by peptide bonds, expressed from a single DNA, and may themselves be chimeric, e.g., may comprise amino acid sequences homologous to portions of at least two different antibody molecules. The BABS are biosynthetic in the sense that they are synthesized in a cellular host made to express a synthetic DNA, that is, a recombinant DNA made by ligation of plural, chemically synthesized oligonucleotides, or by ligation of fragments of DNA derived from the genome of a hybridoma, mature B cell clone, or a cDNA library derived from such natural sources. The single polypeptide chain and linker-containing multifunctional proteins of the invention are properly characterized as "binding sites" in that these synthetic molecules are designed to have specific affinity for a preselected antigenic determinant. The polypeptides of the invention comprise structures which can be patterned after regions of native antibodies known to be responsible for antigen recognition.

### Brief Description of the Drawing

The foregoing and other objects of this invention, the various features thereof, as well as the invention itself, may be more fully understood from the following description, when read together with the accompanying drawings.

Figure 1A is a schematic representation of an intact IgG antibody molecule containing two light chains, each consisting of one variable and one constant domain, and two heavy chains, each consisting of one variable and three constant domains. Figure 1B is a schematic drawing of the structure of Fv proteins (and DNA encoding them) illustrating V_{H} and V_{L} domains, each of which comprises four framework (FR) regions and three complementarity determining (CDR) regions. Boundaries of CDRs are indicated, by way of example, for monoclonal 26-10, a well known and characterized murine monoclonal specific for digoxin.

Figure 2A-2E are schematic representations of some classes of reagents, each of which comprises a biosynthetic antibody binding site.

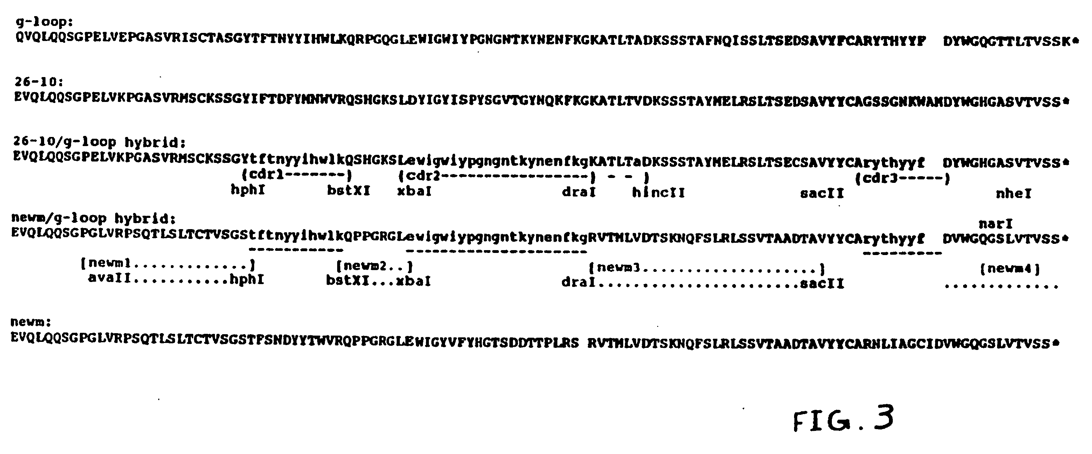

Figure 3 discloses five amino acid sequences (heavy chains) in single letter code lined up vertically to facilitate understanding. Sequence 1 is the known native sequence of V_{H} from murine monoclonal glp-4 (anti-lysozyme). Sequence 2 is the known native sequence of V_{H} from murine monoclonal 26-10 (anti-digoxin). Sequence 3 is a BABS comprising the FRs from 26-10 V_{H} and the CDRs from glp-4 V_{H}. The CDRs are identified in lower case letters; restriction sites in the DNA used to produce chimeric sequence 3 are also identified. Sequence 4 is the known native sequence of V_{H} from human myeloma antibody NEWM. Sequence 5 is a BABS comprising the FRs from NEWM V_{H} and the CDRs from glp-4 V_{H}, i.e., illustrates a "humanized" binding site having a human framework but an affinity for lysozyme similar to murine glp-4.

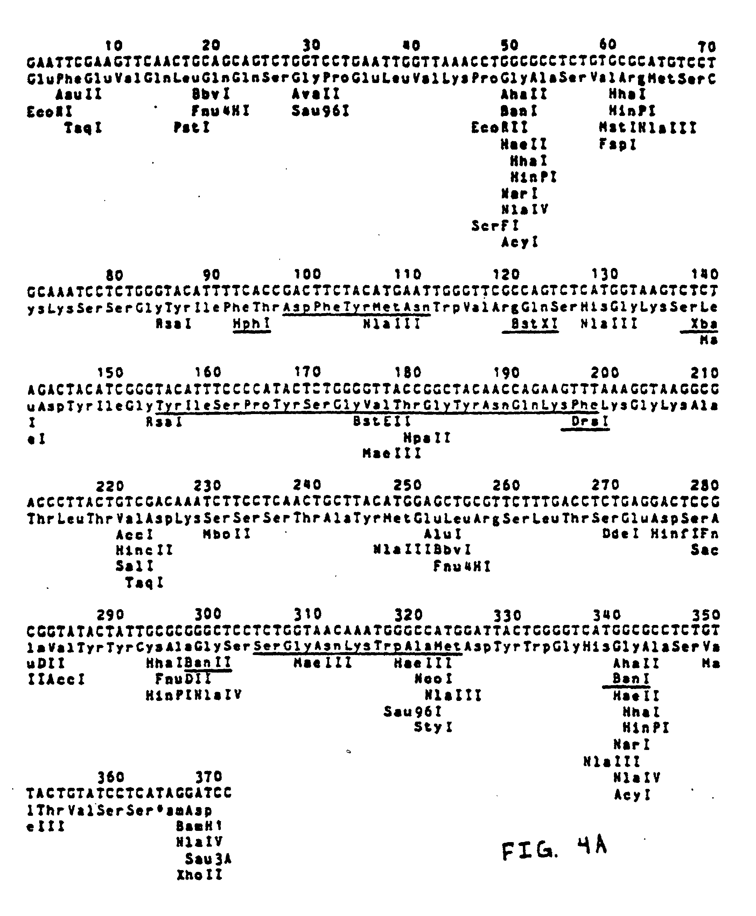

Figures 4A-4F are the synthetic nucleic acid sequences and encoded amino acid sequences of (4A) the heavy chain variable domain of murine anti-digoxin monoclonal 26-10; (4B) the light chain variable domain of murine anti-digoxin monoclonal 26-10; (4C) a heavy chain variable domain of a BABS comprising CDRs of glp-4 and FRs of 26-10; (4D) a light chain variable region of the same BABS; (4E) a heavy chain variable region of a BABS comprising CDRs of glp-4 and FRs of NEWM; and (4F) a light chain variable region comprising CDRs of glp-4 and FRs of NEWM. Delineated are FRs, CDRs, and restriction sites for endonuclease digestion, most of which were introduced during design of the DNA.

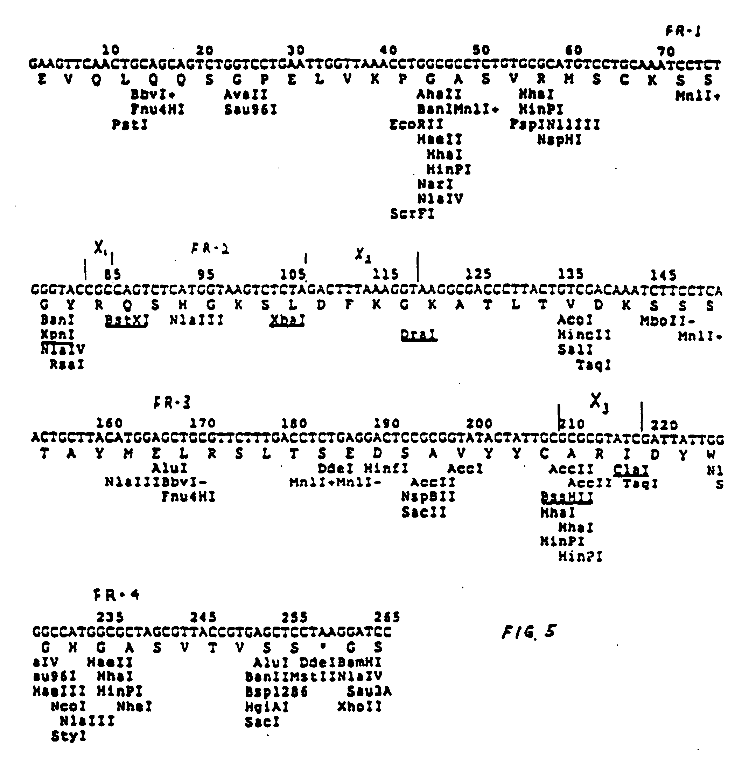

Figure 5 is the nucleic acid and encoded amino acid sequence of a host DNA (V_{H}) designed to facilitate insertion of CDRs of choice. The DNA was designed to have unique 6-base sites directly flanking the CDRs so that relatively small oligonucleotides defining portions of CDRs can be readily inserted, and to have other sites to facilitate manipulation of the DNA to optimize binding properties in a given construct. The framework regions of the molecule correspond to murine FRs (Figure 4A).

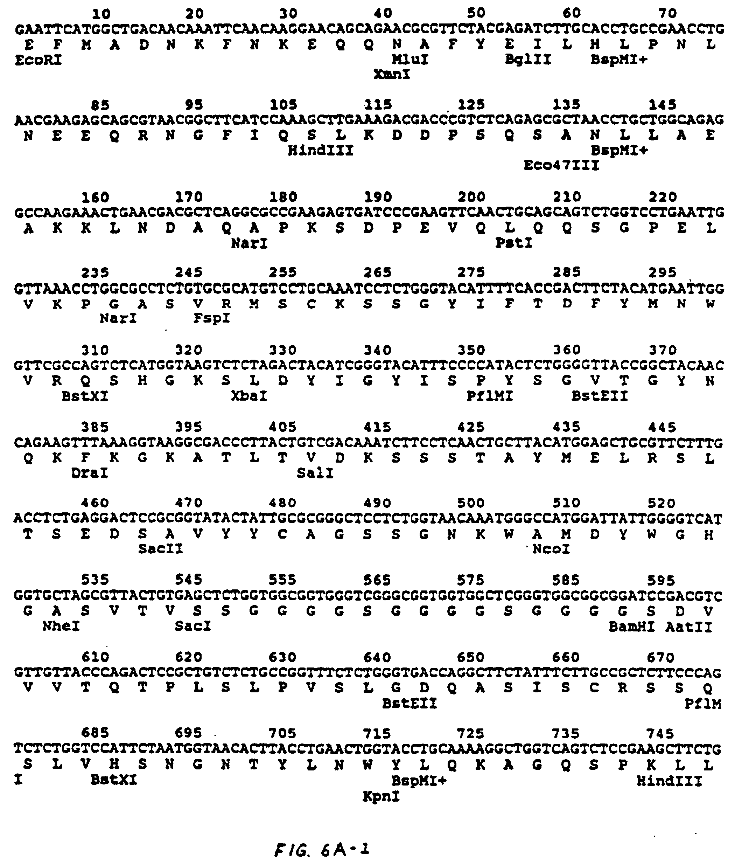

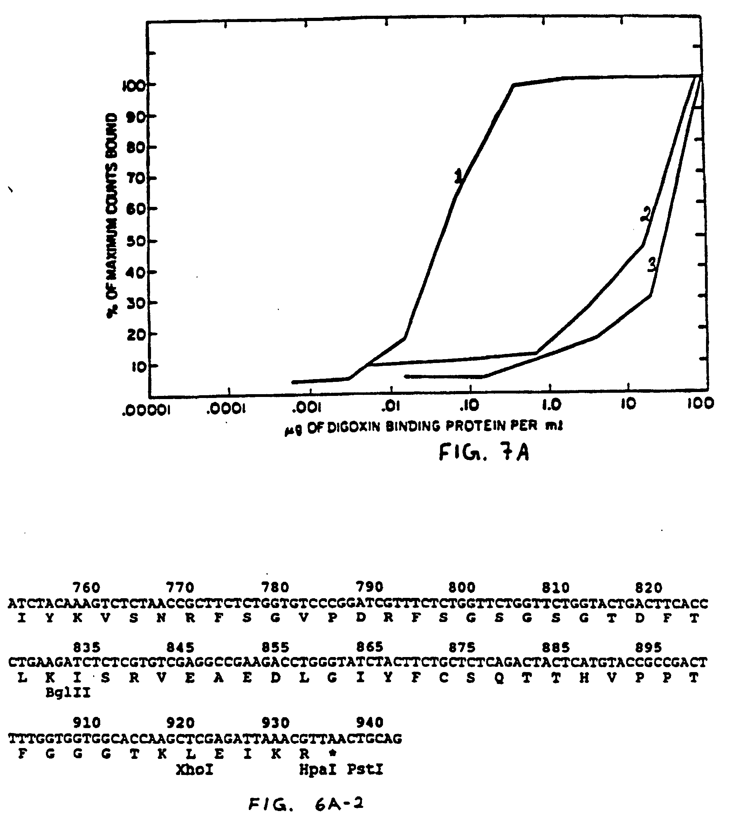

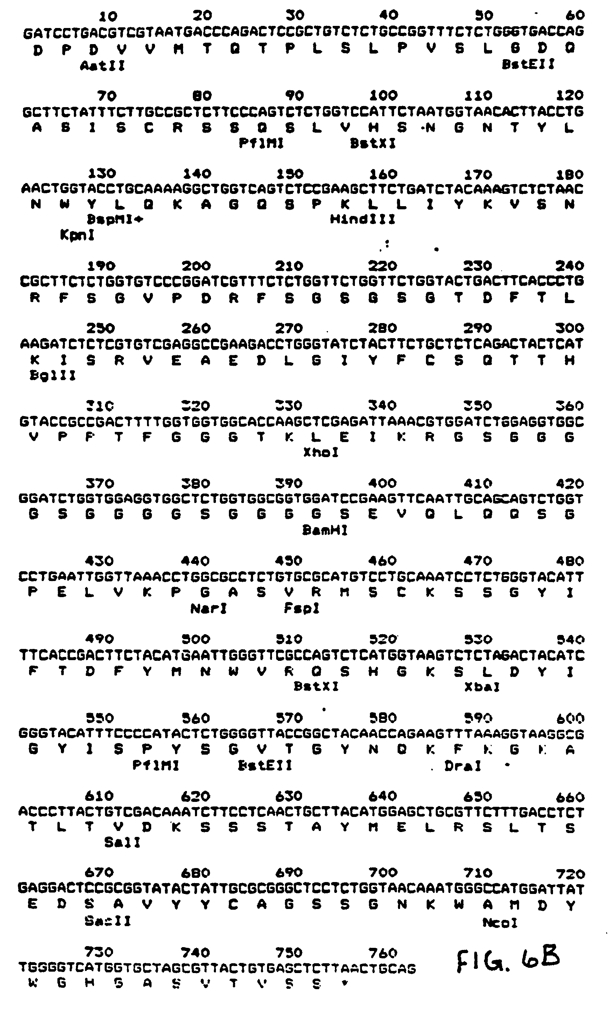

Figures 6A and 6B are multifunctional proteins (and DNA encoding them) comprising a single chain·BASS with the specificity of murine monoclonal 26-10, linked through a spacer to the FB fragment of protein A, here fused as a leader, and constituting a binding site for Fc. The spacer comprises the 11 C-terminal amino acids of the FB followed by Asp-Pro (a dilute acid cleavage site). The single chain BABS comprises sequences mimicking the V_{H} and V_{L} (6A) and the V_{L} and V_{H} (6B) of murine monoclonal 26-10. The V_{L} in construct 6A is altered at residue 4 where valine replaces methionine present in the parent 26-10 sequence. These constructs contain binding sites for both Fc and digoxin. Their structure may be summarized as;

(6A) FB-Asp-Pro-V_{H}-(Gly₄-Ser)₃-V_{L},

and

(6B) FB-Asp-Pro-V_{L}-(Gly₄-Ser)₃-V_{H},

where (Gly₄-Ser)₃ is a polypeptide linker.

In Figures 4A-4E and 6A and 6B, the amino acid sequence of the expression products start after the GAATTC sequences, which codes for an EcoRI splice site, translated as Glu-Phe on the drawings.

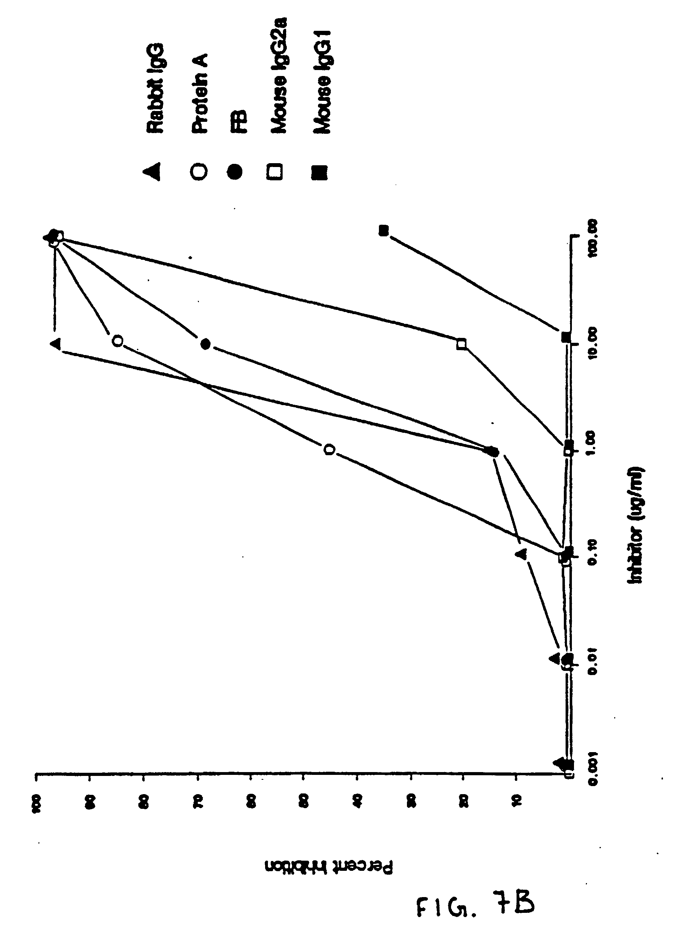

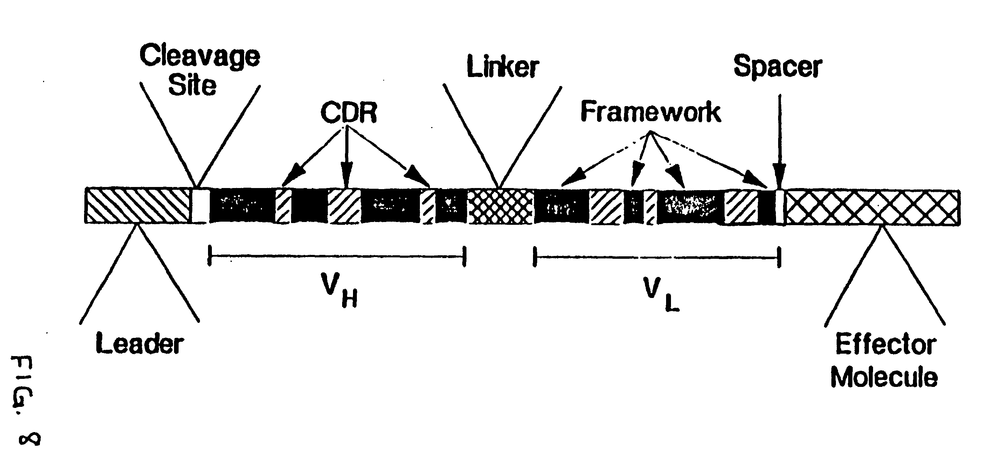

Figure 7A is a graph of percent of maximum counts bound of radioiodinated digoxin versus concentration of binding protein adsorbed to the plate comparing the binding of native 26-10 (curve 1) and the construct of Figure 6A and Figure 2B renatured using two different procedures (curves 2 and 3). Figure 7B is a graph demonstrating the bifunctionality of the FB-(26-10) BABS adhered to microtiter plates through the specific binding of the binding site to the digoxin-BSA coat on the plate. Figure 7B shows the percent inhibition of ¹²⁵I-rabbit-IgG binding to the FB domain of the FB BABS by the addition of IgG, protein A, FB, murine IgG2a, and murine IgGl.

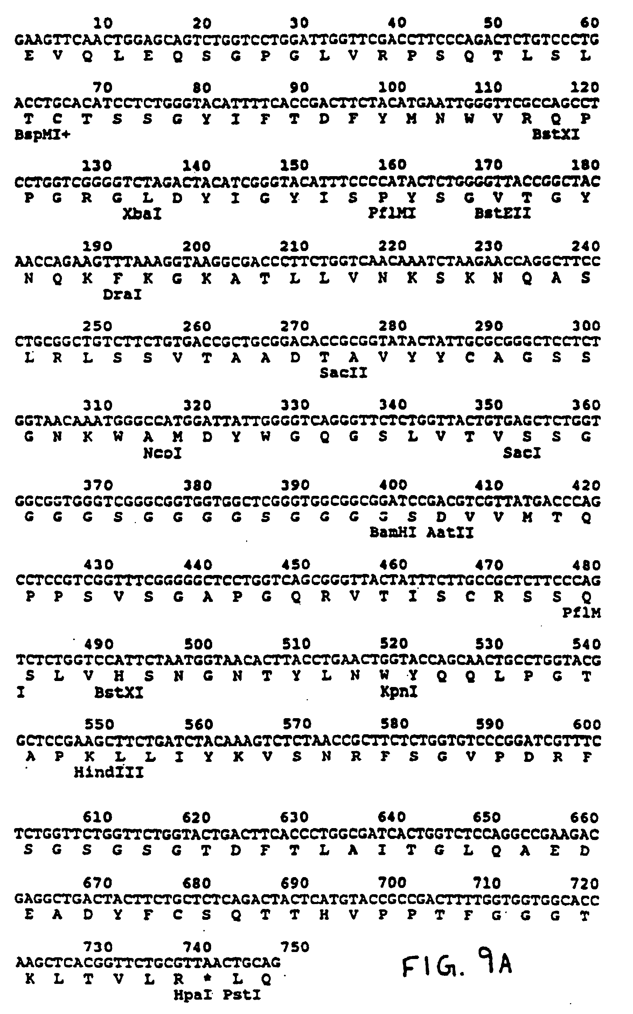

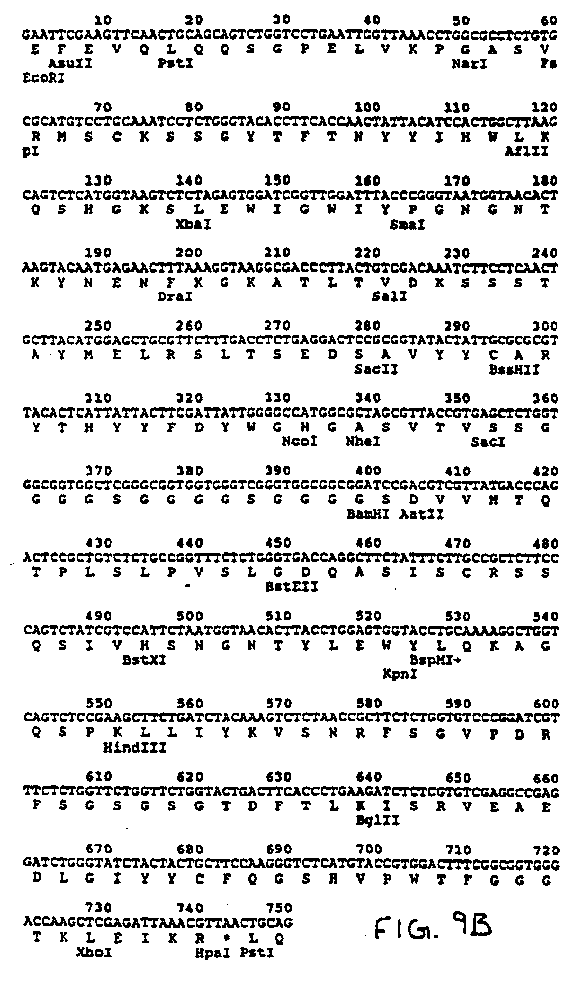

Figure 8 is a schematic representation of a model assembled DNA sequence encoding a multifunctional biosynthetic protein comprising a leader peptide (used to aid expression and thereafter cleaved), a binding site, a spacer, and an effector molecule attached as a trailer sequence.

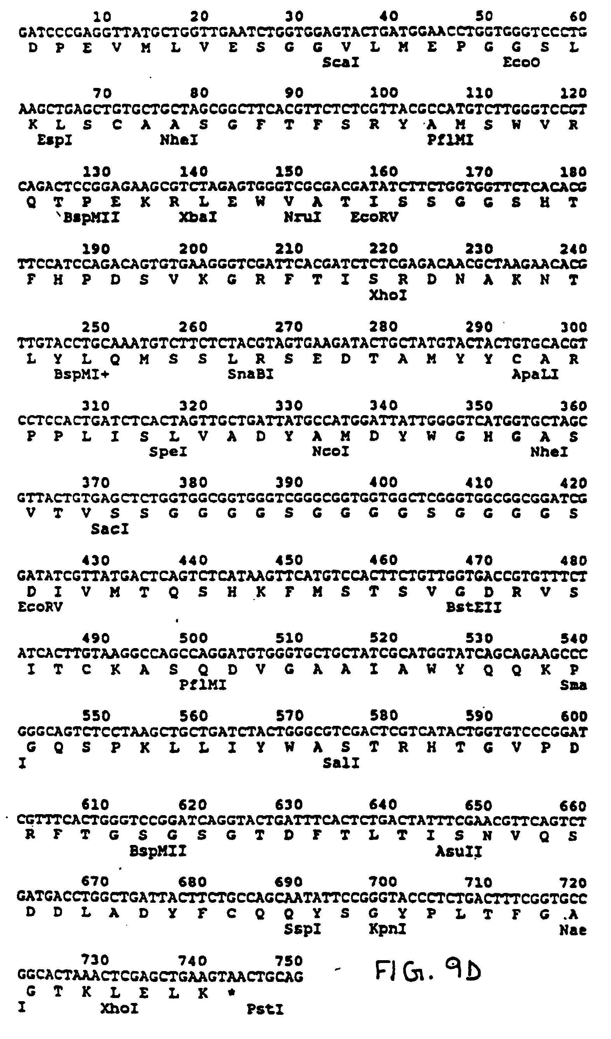

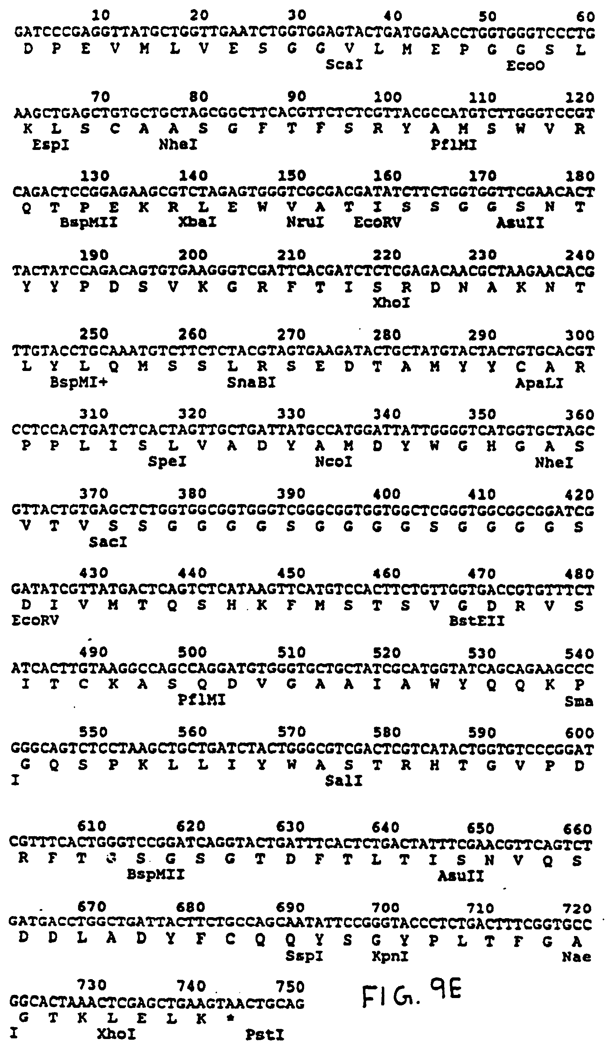

Figure 9A-9E are exemplary synthetic nucleic acid sequences and corresponding encoded amino acid sequences of binding sites of different specificities: (A) FRs from NEWM and CDRs from 26-10 having the digoxin specificity of murine monoclonal 26-10; (B) FRs from 26-10, and CDRs from G-loop-4 (glp-4) having lysozyme specificity; (C) FRs and CDRs from MOPC-315 having dinitrophenol (DNF) specificity; (D) FRs and CDRs from an anti-CEA monoclonal antibody; (E) FRs in both V_{H} and V_{L} and CDR₁ and CDR₃ in V_{H}, and CDR₁, CDR₂, and CDR₃ in V_{L} from an anti-CEA monoclonal antibody; CDR₂ in V_{H} is a CDR₂ consensus sequence found in most immunoglobulin V_{H} regions.

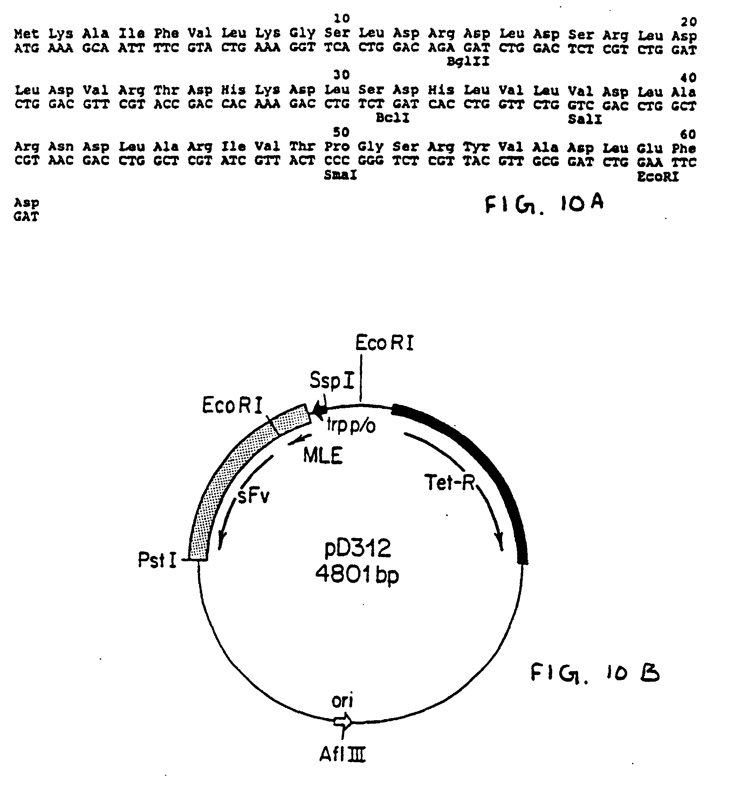

Figure 10A is a schematic representation of the DNA and amino acid sequence of a leader peptide (MLE) protein with corresponding DNA sequence and some major restriction sites. Figure 10B shows the design of an expression plasmid used to express MLE-BABS (26-10). During construction of the gene, fusion partners were joined at the EcoRl site that is shown as part of the leader sequence. The pBR322 plasmid, opened at the unique Sspl and PstI sites, was combined in a 3-part ligation with an SspI to EcoRI fragment bearing the trp promoter and MLE leader and with an EcoRI to PstI fragment carrying the BABS gene. The resulting expression vector confers tetracycline resistance on positive transformants.

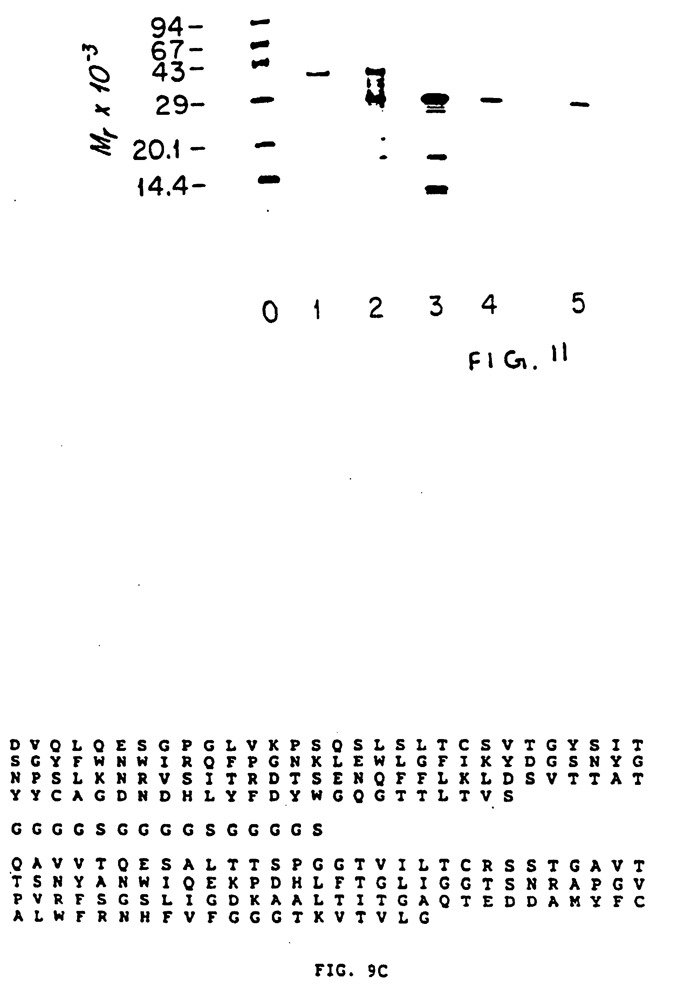

Figure 11 is an SDS-polyacrylamide gel (15%) of the (26-10) BABS at progressive stages of purification. Lane 0 shows low molecular weight standards; lane 1 is the MLE-BABS fusion protein; lane 2 is an acid digest of this material; lane 3 is the pooled DE-52 chromatographed protein; lanes 4 and 5 are the same oubain-Sepharose pool of single chain BABS except that lane 4 protein is reduced and lane 5 protein is unreduced.

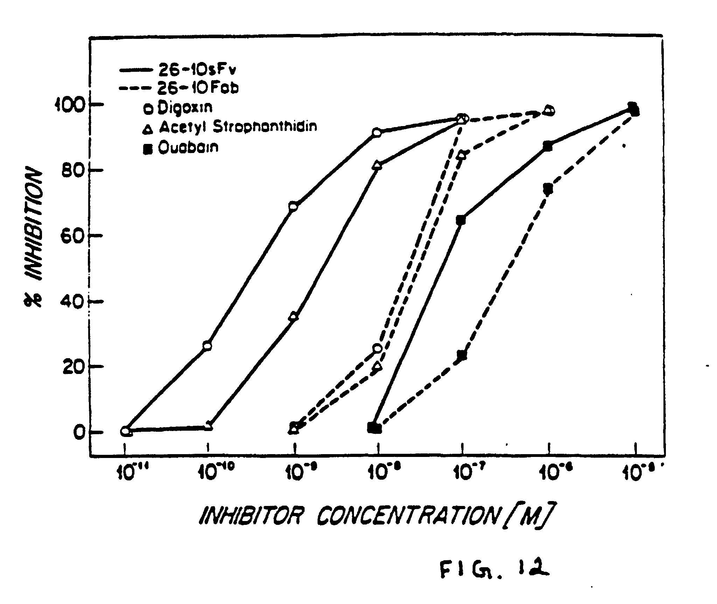

Figure 12 shows inhibition curves for 26-10 BABS and 26-10 Fab species, and indicates the relative,affinities of the antibody fragment for the indicated cardiac glycosides.

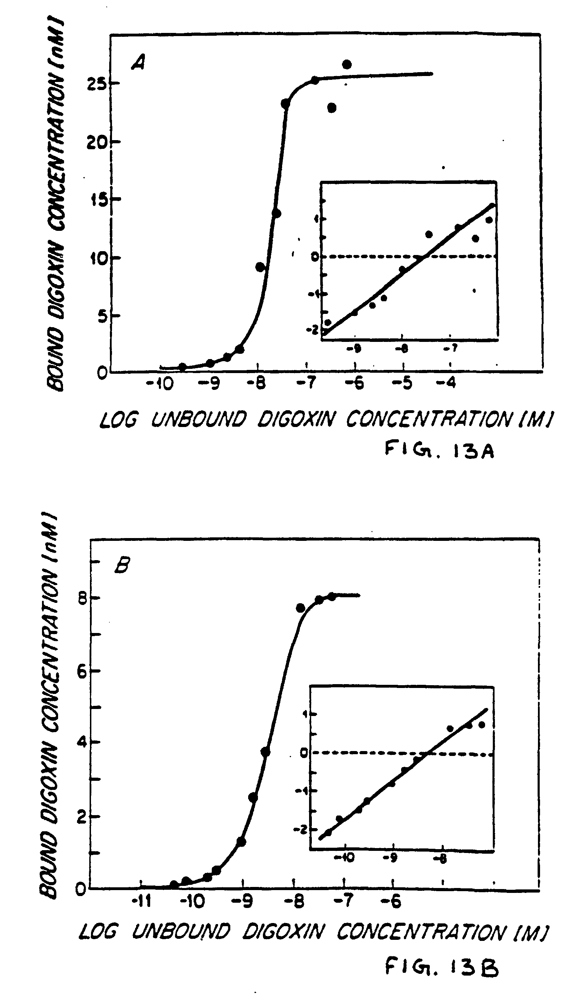

Figures 13A and 13B are plots of digoxin binding curves. (A) shows 26-10 BABS binding isotherm and Sips plot (inset), and (B) shows 26-10 Fab binding isotherm and Sips plot (inset).

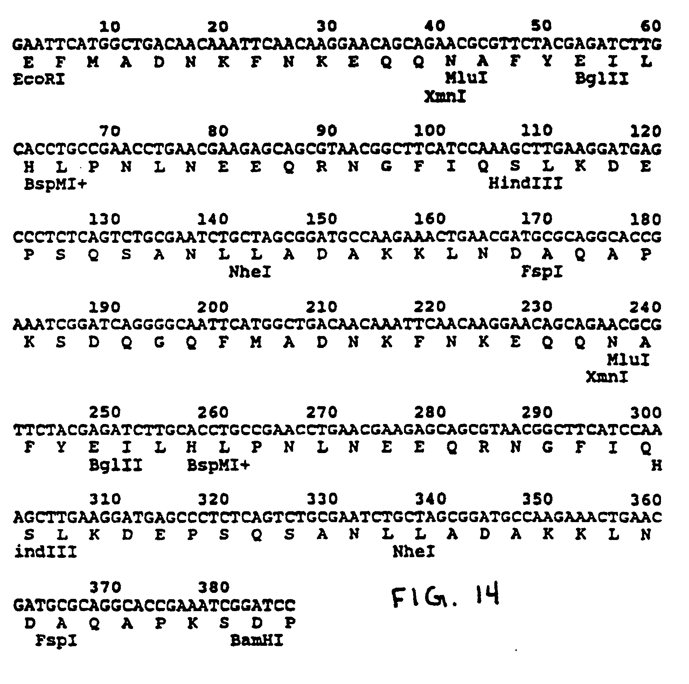

Figure 14 is a nucleic acid sequence and corresponding amino acid sequence of a modified FB dimer leader sequence and various restriction sites.

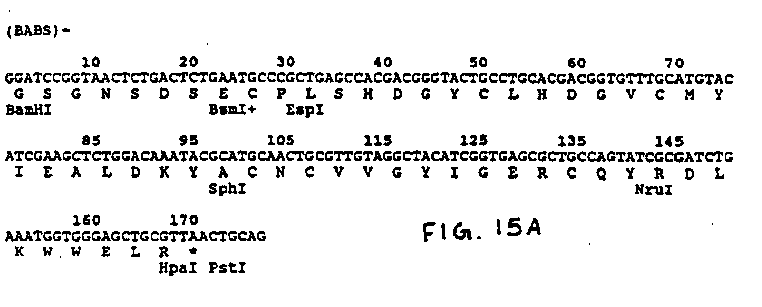

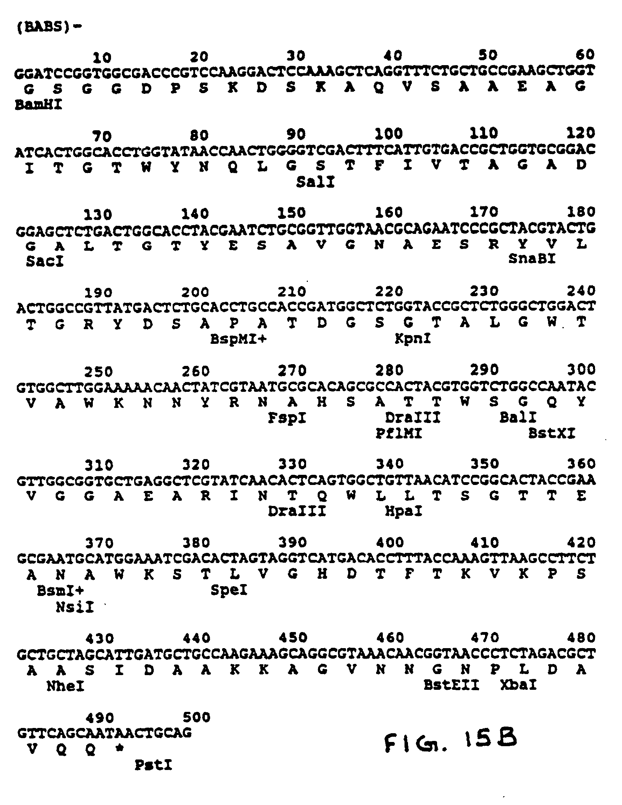

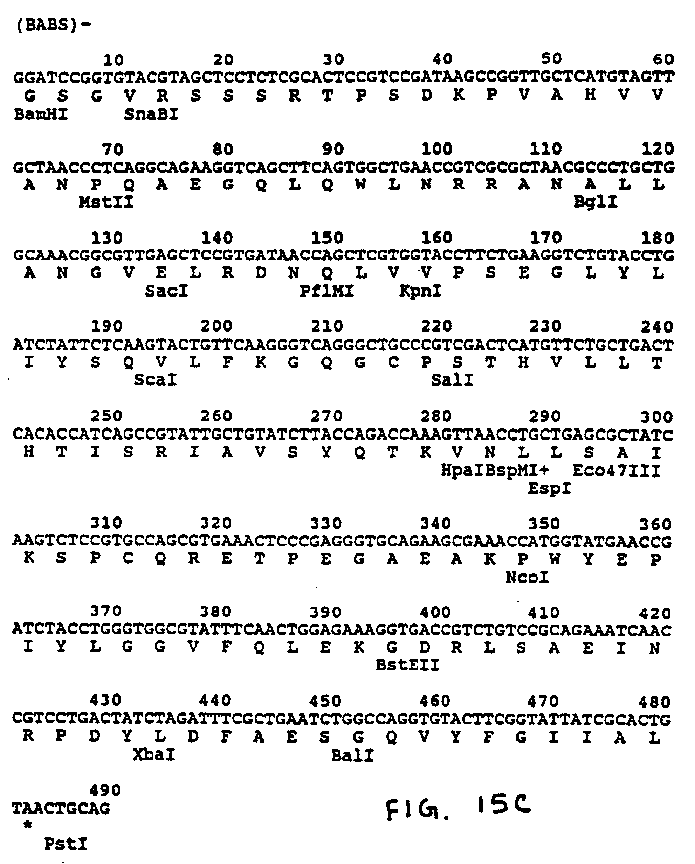

Figure 15A-15H are nucleic acid sequences and corresponding amino acid sequences of biosynthetic multifunctional proteins including a single chain BABS and various biologically active protein trailers linked via a spacer sequence. Also indicated are various endonuclease digestion sites. The trailing sequences are (A) epidermal growth factor (EGF); (B) streptavidin; (C) tumor necrosis factor (TNF); (D) calmodulin; (E) platelet derived growth factor-beta (PDGF-beta); (F) ricin; and (G) interleukin-2, and (H) an FB-FB dimer.

### Description

The invention will first be described in its broadest overall aspects with a more detailed description following.

A class of novel biosynthetic, bi or multifunctional proteins has now been designed and engineered which comprise biosynthetic antibody binding sites, that is, "BABS" or biosynthetic polypeptides defining structure capable of selective antigen recognition and preferential antigen binding, and one or more peptide-bonded additional protein or polypeptide regions designed to have a preselected property. Examples of the second region include amino acid sequences designed to sequester ions, which makes the protein suitable for use as an imaging agent, and sequences designed to facilitate immobilization of the protein for use in affinity chromatography and solid phase immunoassay. Another example of the second region is a bioactive effector molecule, that is, a protein having a conformation suitable for biological activity, such as an enzyme, toxin, receptor, binding site, growth factor, cell differentiation factor, lymphokine, cytokine, hormone, or anti-metabolite. This invention features synthetic, multifunctional proteins comprising these . regions peptide bonded to one or more biosynthetic antibody binding sites, synthetic, single chain proteins designed to bind preselected antigenic determinants with high affinity and specificity, constructs containing multiple binding sites linked together to provide multipoint antigen binding and high net affinity and specificity, DNA encoding these proteins prepared by recombinant techniques, host cells harboring these DNAs, and methods for the production of these proteins and DNAs.

It has also been discovered that biosynthetic domains mimicking the structure of the two chains of an immunoglobulin binding site may be connected by a polypeptide linker while closely approaching, retaining, and often improving their collective binding properties.

The binding site region of the multifunctional proteins comprises at least one, and preferably two domains, each of which has an amino acid sequence homologous to portions of the CDRs of the variable domain of an immunoglobulin light or heavy chain, and other sequence homologous to the FRs of the variable domain of the same, or a second, different immunoglobulin light or heavy chain. The two domain binding site construct also includes a polypeptide linking the domains. Polypeptides so constructed bind a specific preselected antigen determined by the CDRs held in proper conformation by the FRs and the linker. Preferred structures have human FRs, i.e., mimic the amino acid sequence of at least a portion of the framework regions of a human immunoglobulin, and have linked domains which together comprise structure mimicking a V_{H}-V_{L} or V_{L}-V_{H} immunoglobulin two-chain binding site. CDR regions of a mammalian immunoglobulin, such as those of mouse, rat, or human origin are preferred. The biosynthetic antibody binding site comprises FRs homologous with a portion of the FRs of a human immunoglobulin and CDRs homologous with CDRs from a mouse or rat immunoglobulin. This type of chimeric polypeptide displays the antigen binding specificity of the mouse or rat immunoglobulin, while its human framework minimizes human immune reactions. In addition, the chimeric polypeptide may comprise other amino acid sequences. It may comprise, for example, a sequence homologous to a portion of the constant domain of an immunoglobulin, but preferably is free of constant regions (other than FRs).

The binding site region(s) of the chimeric proteins are thus single chain composite polypeptides comprising a structure which in solution behaves like an antibody binding site. The two domain, single chain composite polypeptide has a structure patterned after tandem V_{H} and V_{L} domains, but with the carboxyl terminal of one attached through a linking amino acid sequence to the amino terminal of the other. The linking amino acid sequence may or may not itself be antigenic or biologically active. It preferably spans a distance of at least about 4 nm (40Å), i.e., comprises at least about 14 amino acids, and comprises residues which together present a hydrophilic, relatively unstructured region. Linking amino acid sequences having little or no secondary structure work well. Optionally, one or a pair of unique amino acids or amino acid sequences recognizable by a site specific cleavage agent may be included in the linker. This permits the V_{H} and V_{L}-like domains to be separated after expression, or the linker to be excised after refolding of the binding site.

Either the amino or carboxyl terminal ends (or both ends) of these chimeric, single chain binding sites are attached to an amino acid sequence which itself is bioactive or has some other function to produce a bifunctional or multifunctional protein. For example, the synthetic binding site may include a leader and/or trailer sequence defining a polypeptide having enzymatic activity, independent affinity for an antigen different from the antigen to which the binding site is directed, or having other functions such as to provide a convenient site of attachment for a radioactive ion, or to provide a residue designed to link chemically to a solid support. This fused, independently functional section of protein should be distinguished from fused leaders used simply to enhance expression in prokaryotic host cells or yeasts. The multifunctional proteins also should be distinguished from the "conjugates" disclosed in the prior art comprising antibodies which, after expression, are linked chemically to a second moiety.

Often, a series of amino acids designed as a "spacer" is interposed between the active regions of the multifunctional protein. Use of such a spacer can promote independent refolding of the regions of the protein. The spacer also may include a specific sequence of amino acids recognized by an endopeptidase, for example, endogenous to a target cell (e.g., one having a surface protein recognized by the binding site) so that the bioactive effector protein is cleaved and released at the target. The second functional protein preferably is present as a trailer sequence, as trailers exhibit less of a tendency to interfere with the binding behavior of the BABS.

The therapeutic use of such "self-targeted" bioactive proteins offers a number of advantages over conjugates of immunoglobulin fragments or complete antibody molecules: they are stable, less immunogenic and have a lower molecular weight; they can penetrate body tissues more rapidly for purposes of imaging or drug delivery because of their smaller size; and they can facilitate accelerated clearance of targeted isotopes or drugs. Furthermore, because design of such structures at the DNA level as disclosed herein permits ready selection of bioproperties and specificities, an essentially limitless combination of binding sites and bioactive proteins is possible, each of which can be refined as disclosed herein to optimize independent activity at each region of the synthetic protein. The synthetic proteins can be expressed in procaryotes such as E, coli, and thus are less costly to produce than immunoglobulins or fragments thereof which require expression in cultured animal cell lines.

The invention thus provides a family of recombinant proteins expressed from a single piece of DNA, all of which have the capacity to bind specifically with a predetermined antigenic determinant. The preferred species of the proteins comprise a second domain which functions independently of the binding region. In this aspect the invention provides an array of "self-targeted" proteins which have a bioactive function and which deliver that function to a locus determined by the binding site's specificity. It also provides biosynthetic binding proteins having attached polypeptides suitable for attachment to immobilization matrices which may be used in affinity chromatography and solid phase immunoassay applications, or suitable for attachment to ions, e.g., radioactive ions, which may be used for in vivo imaging.

The successful design and manufacture of the proteins of the invention depends on the ability to produce biosynthetic binding sites, and most preferably, sites comprising two domains mimicking the variable domains of immunoglobulin connected by a linker.

As is now well known, Fv, the minimum antibody fragment which contains a complete antigen recognition and binding site, consists of a dimer of one heavy and one light chain variable domain in noncovalent association (Figure 1A). It is in this configuration that the three complementarity determining regions of each variable domain interact to define an antigen binding site on the surface of the V_{H}-V_{L} dimer. Collectively, the six complementarity determining regions (see Figure 1B) confer antigen binding specificity to the antibody. FRs flanking the CDRs have a tertiary structure which is essentially conserved in native immunoglobulins of species as diverse as human and mouse. These FRs serve to hold the CDRs in their appropriate orientation. The constant domains are not required for binding function, but may aid in stabilizing V_{H}-V_{L} interaction. Even a single variable domain (or half of an Fv comprising only three CDRs specific for an antigen) has the ability to recognize and bind antigen, although at a lower affinity than an entire binding site (Painter et al. (1972) Biochem. 11:1327-1337).

This knowledge of the structure of immunoglobulin proteins has now been exploited to develop multifunctional fusion proteins comprising biosynthetic antibody binding sites and one or more other domains.

The structure of these biosynthetic proteins in the region which impart the binding properties to the protein is analogous to the Fv region of a natural antibody. It comprises at least one, and preferably two domains consisting of amino acids defining V_{H} and V_{L}-like polypeptide segments connected by a linker which together form the tertiary molecular structure responsible for affinity and specificity. Each domain comprises a set of amino acid sequences analogous to immunoglobulin CDRs held in appropriate conformation by a set of sequences analogous to the framework regions (FRs) of an Fv fragment of a natural antibody.

The term CDR, as used herein, refers to amino acid sequences which together define the binding affinity and specificity of the natural Fv region of a native immunoglobulin binding site, or a synthetic polypeptide which mimics this function. CDRs typically are not wholly homologous to hypervariable regions of natural Fvs, but rather also may include specific amino acids or amino acid sequences which flank the hypervariable region and have heretofore been considered framework not directly determinitive of complementarity. The term FR, as used herein, refers to amino acid sequences flanking or interposed between CDRs.

The CDR and FR polypeptide segments are designed based on sequence analysis of the Fv region of preexisting antibodies or of the DNA encoding them. In one embodiment, the amino acid sequences constituting the FR regions of the BABS are analogous to the FR sequences of a first preexisting antibody, for example, a human IgG. The amino acid sequences constituting the CDR regions are analogous to the sequences from a second, different preexisting antibody, for example, the CDRs of a murine IgG. Alternatively, the CDRs and FRs from a single preexisting antibody from, e.g., an unstable or hard to culture hybridoma, may be copied in their entirety.

The design and biosynthesis of various reagents is enabled, all of which are characterized by a region having affinity for a preselected antigenic determinant. The binding site and other regions of the biosynthetic protein are designed with the particular planned utility of the protein in mind. Thus, if the reagent is designed for intravascular use in mammals, the FR regions may comprise amino acids similar or identical to at least a portion of the framework region amino acids of antibodies native to that mammalian species. On the other hand, the amino acids comprising the CDRs may be analogous to a portion of the amino acids from the hypervariable region (and certain flanking amino acids) of an antibody having a known affinity and specificity, e.g., a murine or rat monoclonal antibody.

Other sections of native immunoglobulin protein structure, e.g., C_{H} and C_{L}, need not be present and normally are intentionally omitted from the biosynthetic proteins. However, the proteins of the invention normally comprise additional polypeptide or protein regions defining a bioactive region, e.g., a toxin or enzyme, or a site onto which a toxin or a remotely detectable substance can be attached.

The invention thus can provide intact biosynthetic antibody binding sites analogous to V_{H}-V_{L} dimers, either non-covalently associated, disulfide bonded, or preferably linked by a polypeptide sequence to form a composite V_{H}-V_{L} or V_{L}-V_{H} polypeptide which may be essentially free of antibody constant region. The invention also provides proteins analogous to an independent V_{H} or V_{L} domain, or dimers thereof. Any of these proteins may be provided in a form linked to, for example, amino acids analogous or homologous to a bioactive molecule such as a hormone or toxin.

Connecting the independently functional regions of the protein is a spacer comprising a short amino acid sequence whose function is to separate the functional regions so that they can independently assume their active tertiary conformation. The spacer can consist of an amino acid sequence present on the end of a functional protein which sequence is not itself required for its function, and/or specific sequences engineered into the protein at the DNA level.

The spacer generally may comprise between 5 and 25 residues. Its optimal length may be determined using constructs of different spacer lengths varying, for example, by units of 5 amino acids. The specific amino acids in the spacer can vary. Cysteines should be avoided. Hydrophilic amino acids are preferred. The spacer sequence may mimic the sequence of a hinge region of an immunoglobulin. It may also be designed to assume a structure, such as a helical structure. Proteolytic cleavage sites may be designed into the spacer separating the variable region-like sequences from other pendant sequences so as to facilitate cleavage of intact BABS, free of other protein, or so as to release the bioactive protein in vivo.

Figures 2A-2E illustrate five examples of protein structures embodying the single polypeptide chain and linkers of the invention that can be produced by following the teaching disclosed herein. All of these examples are characterized by a biosynthetic polypeptide defining a binding site 3, comprising amino acid sequences comprising CDRs and FRs, often derived from different immunoglobulins, or sequences homologous to a portion of CDRs and FRs from different immunoglobulins. Figure 2A depicts a single chain construct comprising a polypeptide domain 10 having an amino acid sequence analogous to the variable region of an immunoglobulin heavy chain, bound through its carboxyl end to a polypeptide linker 12, which in turn is bound to a polypeptide domain 14 having an amino acid sequence analogous to the variable region of an immunoglobulin light chain. Of course, the light and heavy chain domains may be in reverse order. Alternatively, the binding site may comprise two substantially homologous amino acid sequences which are both analogous to the variable region of an immunoglobulin heavy or light chain.

The linker 12 should be long enough (e.g., about 15 amino acids or about 4 nm (40 Å) to permit the chains 10 and 14 to assume their proper conformation. The linker 12 may comprise an amino acid sequence homologous to a sequence identified as "self" by the species into which it will be introduced, if drug use is intended. For example, the linker may comprise an amino acid sequence patterned after a hinge region of an immunoglobulin. The linker preferably comprises hydrophilic amino acid sequences. It may also comprise a bioactive polypeptide such as a cell toxin which is to be targeted by the binding site, or a segment easily labelled by a radioactive reagent which is to be delivered, e.g., to the site of a tumor comprising an epitope recognized by the binding site. The linker may also include one or two built-in cleavage sites, i.e., an amino acid or amino acid sequence susceptible to attack by a site specific cleavage agent as described below. This strategy permits the V_{H} and V_{L}-like domains to be separated after expression, or the linker to be excised after folding while retaining the binding site structure in non-covalent association. The amino acids of the linker preferably are selected from among those having relatively small, unreactive side chains. Alanine, serine, and glycine are preferred.

Generally, the design of the linker involves considerations similar to the design of the spacer, excepting that binding properties of the linked domains are seriously degraded if the linker sequence is shorter than about 2 nm (20Å) in length, i.e., comprises less than about 10 residues. Linkers longer than the approximate 4 nm (40Å) distance between the N terminal of a native variable region and the C-terminal of its sister chain may be used, but also potentially can diminish the BABS binding properties. Linkers comprising between 12 and 18 residues are preferred. The preferred length in specific constructs may be determined by varying linker length first by units of 5 residues, and second by units of 1-4 residues after determining the best multiple of the pentameric starting units.

Additional proteins or polypeptides may be attached to either or both the amino or carboxyl termini of the binding site to produce multifunctional proteins of the type illustrated in Figures 2B-2E. As an example, in Figure 2B, a helically coiled'polypeptide structure 16 comprises a protein A fragment (FB) linked to the amino terminal end of a V_{H}-like domain 10 via a spacer 18. Figure 2C illustrates a bifunctional protein having an effector polypeptide 20 linked via spacer 22 to the carboxyl terminus of polypeptide 14 of binding protein segment 2. This effector polypeptide 20 may consist of, for example, a toxin, therapeutic drug, binding protein, enzyme or enzyme fragment, site of attachment for an imaging agent (e.g., to chelate a radioactive ion such as indium), or site of selective attachment to an immobilization matrix so that the BABS can be used in affinity chromatography or solid phase binding assay. This effector alternatively may be linked to the amino terminus of polypeptide 10, although trailers are preferred. Figure 2D depicts a trifunctional protein comprising a linked pair of BABS 2 having another distinct protein domain 20 attached to the N-terminus of the first binding protein segment. Use of multiple BABS in a single protein enables production of constructs having very high selective affinity for multiepitopic sites such as cell surface proteins.

The independently functional domains are attached by a spacer 18 (Figs 2B and 2D) covalently linking the C terminus of the protein 16 or 20 to the N-terminus of the first domain 10 of the binding protein segment 2, or by a spacer 22 linking the C-terminus of the second binding domain 14 to the N-terminus of another protein (Figs. 2C and 2D). The spacer may be an amino acid sequence analogous to linker sequence 12, or it may take other forms. As noted above, the spacer's primary function is to separate the active protein regions to promote their independent bioactivity and permit each region to assume its bioactive conformation independent of interference from its neighboring structure.

Figure 2E depicts another type of reagent, comprising a BABS having only one set of three CDRs, e.g., analogous to a heavy chain variable region, which retains a measure of affinity for the antigen. Attached to the carboxyl end of the polypeptide 10 or 14 comprising the FR and CDR sequences constituting the binding site 3 through spacer 22 is effector polypeptide 20 as described above.

As is evidenced from the foregoing, the invention provides a large family of reagents comprising proteins, at least a portion of which defines a binding site patterned after the variable region of an immunoglobulin. It will be apparent that the nature of any protein fragments linked to the BABS, and used for reagents embodying the invention, are essentially unlimited, the essence of the invention being the provision, either alone or linked to other proteins, of binding sites having specificities to any antigen desired.

The clinical administration of multifunctional proteins comprising a BABS, or a BABS alone, affords a number of advantages over the use of intact natural or chimeric antibody molecules, fragments thereof, and conjugates comprising such antibodies linked chemically to a second bioactive moiety. The multifunctional proteins described herein offer fewer cleavage sites to circulating proteolytic enzymes, their functional domains are connected by peptide bonds to polypeptide linker or spacer sequences, and thus the proteins have improved stability. Because of their smaller size and efficient design, the multifunctional proteins described herein reach their target tissue more rapidly, and are cleared more quickly from the body. They also have reduced immunogenicity. In addition, their design facilitates coupling to other moieties in drug targeting and imaging application. Such coupling may be conducted chemically after expression of the BABS to a site of attachment for the coupling product engineered into the protein at the DNA level. Active effector proteins having toxic, enzymatic, binding, modulating, cell differentiating, hormonal, or other bioactivity are expressed from a single DNA as a leader and/or trailer sequence, peptide bonded to the BABS.

### Design and Manufacture

The single polypeptide chain and linkers of the invention are designed at the DNA level. The chimeric or synthetic DNAs are then expressed in a suitable host system, and the expressed proteins are collected and renatured if necessary. A preferred general structure of the DNA encoding the proteins is set forth in Figure 8. As illustrated, it encodes an optimal leader sequence used to promote expression in procaryotes having a built-in cleavage site recognizable by a site specific cleavage agent, for example, an endopeptidase, used to remove the leader after expression. This is followed by DNA encoding a V_{H}-like domain, comprising CDRs and FRs, a linker, a V_{L}-like domain, again comprising CDRs and FRs, a spacer, and an effector protein. After expression, folding, and cleavage of the leader, a bifunctional protein is produced having a binding region whose specificity is determined by the CDRs, and a peptide-linked independently functional effector region.

The ability to design the BABS of the invention depends on the ability *to* determine the sequence of the amino acids in the variable region of monoclonal antibodies of interest, or the DNA encoding them. Hybridoma technology enables production of cell lines secreting antibody to essentially any desired substance that produces an immune response. RNA encoding the light and heavy chains of the immunoglobulin can then be obtained from the cytoplasm of the hybridoma. The 5' end portion of the mRNA can be used to prepare cDNA for subsequent sequencing, or the amino acid sequence of the hypervariable and flanking framework regions can be determined by amino acid sequencing of the V region fragments of the H and L chains. Such sequence analysis is now conducted routinely. This knowledge, coupled with observations and deductions of the generalized structure of immunoglobulin Fvs, permits one to design synthetic genes encoding FR and CDR sequences which likely will bind the antigen. These synthetic genes are then prepared using known techniques, or using the technique disclosed below, inserted into a suitable host, and expressed, and the expressed protein is purified. Depending on the host cell, renaturation techniques may be required to attain proper conformation. The various proteins are then tested for binding ability, and one having appropriate affinity is selected for incorporation into a reagent of the type described above. If necessary, point substitutions seeking to optimize binding may be made in the DNA using conventional casette mutagenesis or other protein engineering methodology such as is disclosed below.

Preparation of the proteins of the invention also is dependent on knowledge of the amino acid sequence (or corresponding DNA or RNA sequence) of bioactive proteins such as enzymes, toxins, growth factors, cell differentiation factors, receptors, anti-metabolites, hormones or various cytokines or lymphokines. Such sequences are reported in the literature and available through computerized data banks.

The DNA sequences of the binding site and the second protein domain are fused using conventional techniques, or assembled from synthesized oligonucleotides, and then expressed using equally conventional techniques.

The processes for manipulating, amplifying, and recombining DNA which encode amino acid sequences of interest are generally well known in the art, and therefore, not described in detail herein. Methods of identifying and isolating genes encoding antibodies of interest are well understood, and described in the patent and other literature. In general, the methods involve selecting genetic material coding for amino acids which define the proteins of interest, including the CDRs and FRs of interest, according to the genetic code.

Accordingly, the construction of DNAs encoding proteins as disclosed herein can be done using known techniques involving the use of various restriction enzymes which make sequence specific cuts in DNA to produce blunt ends or cohesive ends, DNA ligases, techniques enabling enzymatic addition of sticky ends to blunt-ended DNA, construction of synthetic DNAs by assembly of short or medium length oligonucleotides, cDNA synthesis techniques, and synthetic probes for isolating immunoglobulin or other bioactive protein genes. Various promoter sequences and other regulatory DNA sequences used in achieving expression, and various types of host cells are also known and available. Conventional transfection techniques, and equally conventional techniques for cloning and subcloning DNA are useful in the practice of this invention and known to those skilled in the art. Various types of vectors may be used such as plasmids and viruses including animal viruses and bacteriophages. The vectors may exploit various marker genes which impart to a successfully transfected cell a detectable phenotypic property that can be used to identify which of a family of clones has successfully incorporated the recombinant DNA of the vector.

One method for obtaining DNA encoding the proteins disclosed herein is by assembly of synthetic oligonucleotides produced in a conventional, automated, polynucleotide synthesizer followed by ligation with appropriate ligases. For example, overlapping, complementary DNA fragments comprising 15 bases may be synthesized semi manually using phosphoramidite chemistry, with end segments left unphosphorylated to prevent polymerization during ligation. One end of the synthetic DNA is left with a "sticky end" corresponding to the site of action of a particular restriction endonuclease, and the other end is left with an end corresponding to the site of action of another restriction endonuclease. Alternatively, this approach can be fully automated. The DNA encoding the protein may be created by synthesizing longer single strand fragments (e.g., 50-100 nucleotides long) in, for example, a Biosearch oligonucleotide synthesizer, and then ligating the fragments.

A method of producing BABS is to produce a synthetic DNA encoding a polypeptide comprising, e.g., human FRs, and intervening "dummy" CDRs, or amino acids having no function except to define suitably situated unique restriction sites. This synthetic DNA is then altered by DNA replacement, in which restriction and ligation is employed to insert synthetic oligonucleotides encoding CDRs defining a desired binding specificity in the proper location between the FRs. This approach facilitates empirical refinement of the binding properties of the BABS.

This technique is dependent upon the ability to cleave a DNA corresponding in structure to a variable domain gene at specific sites flanking nucleotide sequences encoding CDRs. These restriction sites in some cases may be found in the native gene. Alternatively, non-native restriction sites may be engineered into the nucleotide sequence resulting in a synthetic gene with a different sequence of nucleotides than the native gene, but encoding the same variable region amino acids because of the degeneracy of the genetic code. The fragments resulting from endonuclease digestion, and comprising FR-encoding sequences, are then ligated to non-native CDR-encoding sequences to produce a synthetic variable domain gene with altered antigen binding specificity. Additional nucleotide sequences encoding, for example, constant region amino acids or a bioactive molecule may then be linked to the gene sequences to produce a bifunctional protein.

The expression of these synthetic DNA's can be achieved in both prokaryotic and eucaryotic systems via transfection with an appropriate vector. In E. coli and other microbial hosts, the synthetic genes can be expressed as fusion protein which is subsequently cleaved. Expression in eucaryotes can be accomplished by the transfection of DNA sequences encoding CDR and FR region amino acids and the amino acids defining a second function into a myeloma or other type of cell line. By this strategy intact hybrid antibody molecules having hybrid Fv regions and various bioactive proteins including a biosynthetic binding site may be produced. For fusion protein expressed in bacteria, subsequent proteolytic cleavage of the isolated fusions can be performed to yield free BABS, which can be renatured to obtain an intact biosynthetic, hybrid antibody binding site.

Heretofore, it has not been possible to cleave the heavy and light chain region to separate the variable and constant regions of an immunoglobulin so as to produce intact Fv, except in specific cases not of commercial utility. However, one method of producing BABS is to redesign DNAs encoding the heavy and light chains of an immunoglobulin, optionally altering its specificity or humanizing its FRs, and incorporating a cleavage site and "hinge region" between the variable and constant regions of both the heavy and light chains. Such chimeric antibodies can be produced in transfectomas or the like and subsequently cleaved using a preselected endopeptidase.

The hinge region is a sequence of amino acids which serve to promote efficient cleavage by a preselected cleavage agent at a preselected, built-in cleavage site. It is designed to promote cleavage preferentially at the cleavage site when the polypeptide is treated with the cleavage agent in an appropriate environment.

The hinge region can take many different forms. Its design involves selection of amino acid residues (and a DNA fragment encoding them) which impart to the region of the fused protein about the cleavage site an appropriate polarity, charge distribution, and stereochemistry which, in the aqueous environment where the cleavage takes place, efficiently exposes the cleavage site to the cleavage agent in preference to other potential cleavage sites that may be present in the polypeptide, and/or to improve the kinetics of the cleavage reaction. In specific cases, the amino acids of the hinge are selected and assembled in sequence based on their known properties, and then the fused polypeptide sequence is expressed, tested, and altered for refinement.

The hinge region is free of cysteine. This enables the cleavage reaction to be conducted under conditions in which the protein assumes its tertiary conformation, and may be held in this conformation by intramolecular disulfide bonds. It has been discovered that in these conditions access of the protease to potential cleavage sites which may be present within the target protein is hindered. The hinge region may comprise an amino acid sequence which includes one or more proline residues. This allows formation of a substantially unfolded molecular segment. Aspartic acid, glutamic acid, arginine, lysine, serine, and threonine residues maximize ionic interactions and may be present in amounts and/or in sequence which renders the moiety comprising the hinge water soluble.

The cleavage site preferably is immediately adjacent the Fv polypeptide chains and comprises one amino acid or a sequence of amino acids exclusive of any sequence found in the amino acid structure of the chains in the Fv. The cleavage site preferably is designed for unique or preferential cleavage by a specific selected agent. Endopeptidases are preferred, although non-enzymatic (chemical) cleavage agents may be used. Many useful cleavage agents, for instance, cyanogen bromide, dilute acid, trypsin, Staphylococcus aureus V-8 protease, post proline cleaving enzyme, blood coagulation Factor Xa, enterokinase, and renin, recognize and preferentially or exclusively cleave particular cleavage sites. One currently preferred cleavage agent is V-8 protease. The currently preferred cleavage site is a Glu residue. Other useful enzymes recognize multiple residues as a cleavage site, e.g., factor Xa (Ile-Glu-Gly-Arg) or enterokinase (Asp-Asp-Asp-Asp-Lys). The principles of this selective cleavage approach may also be used in the design of the linker and spacer sequences of the multifunctional constructs of the invention where an exciseable linker or selectively cleavable linker or spacer is desired.

### Design of Synthetic V_{H} and V_{L} Mimics

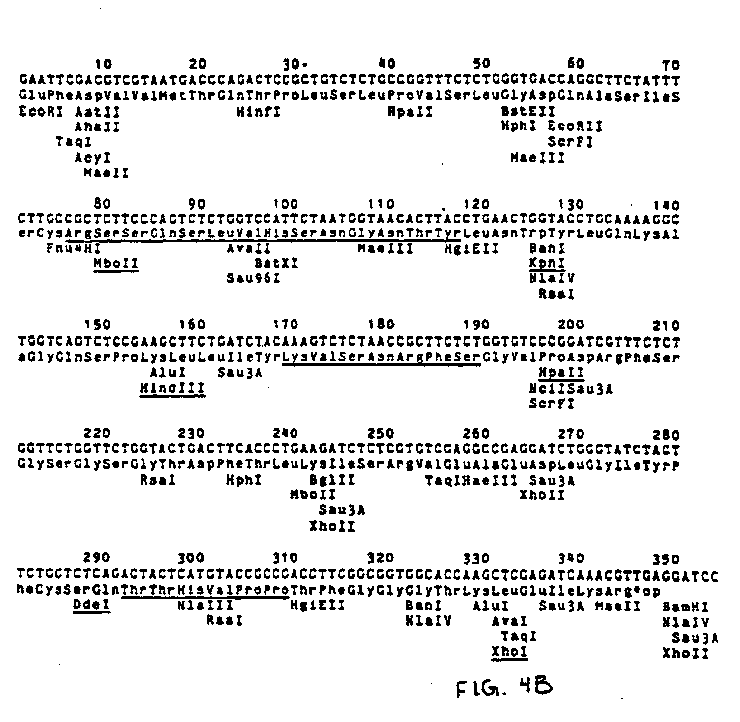

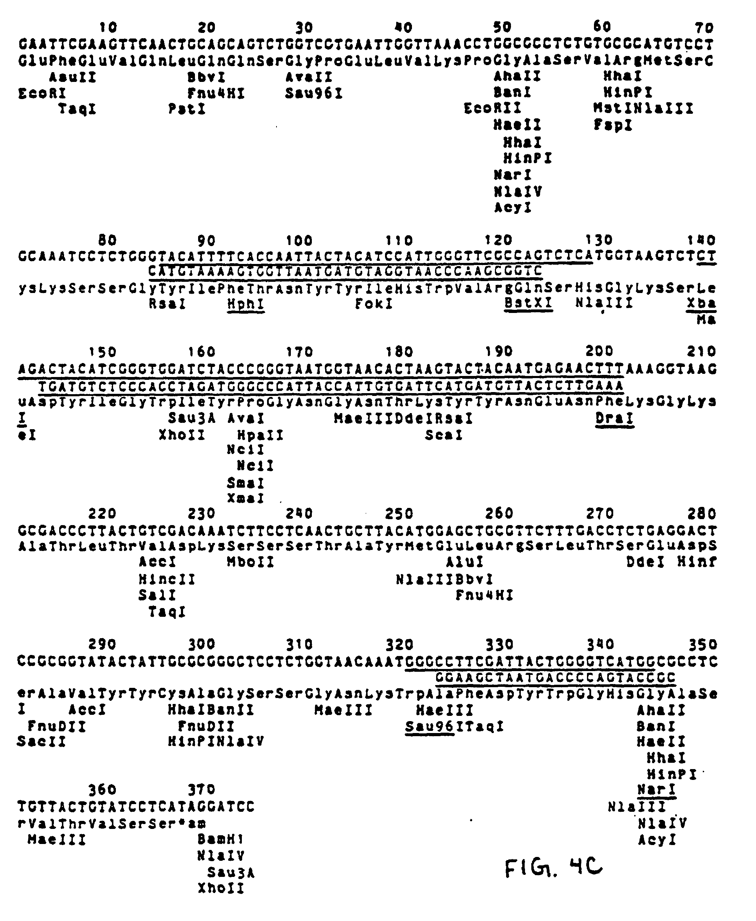

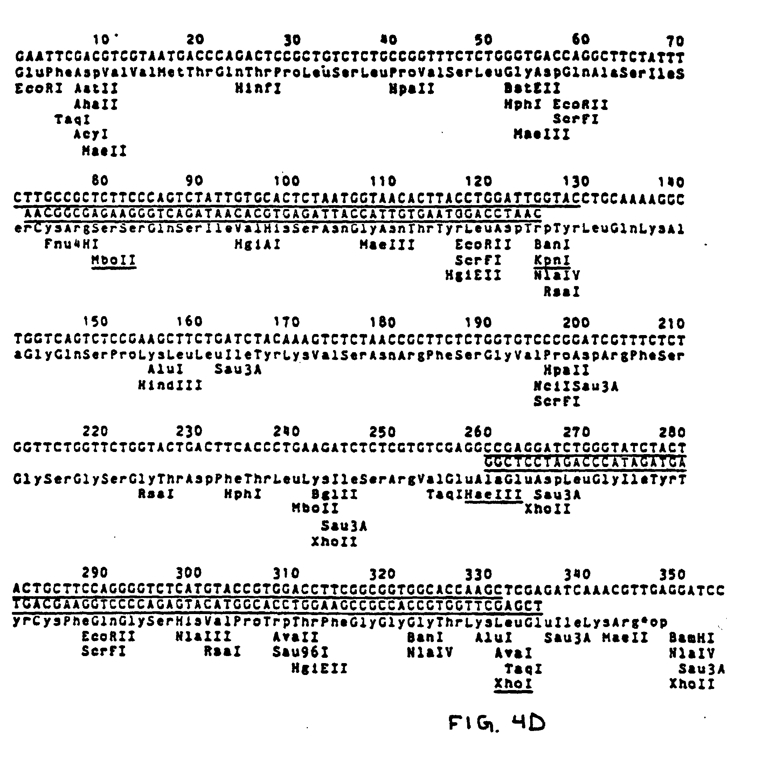

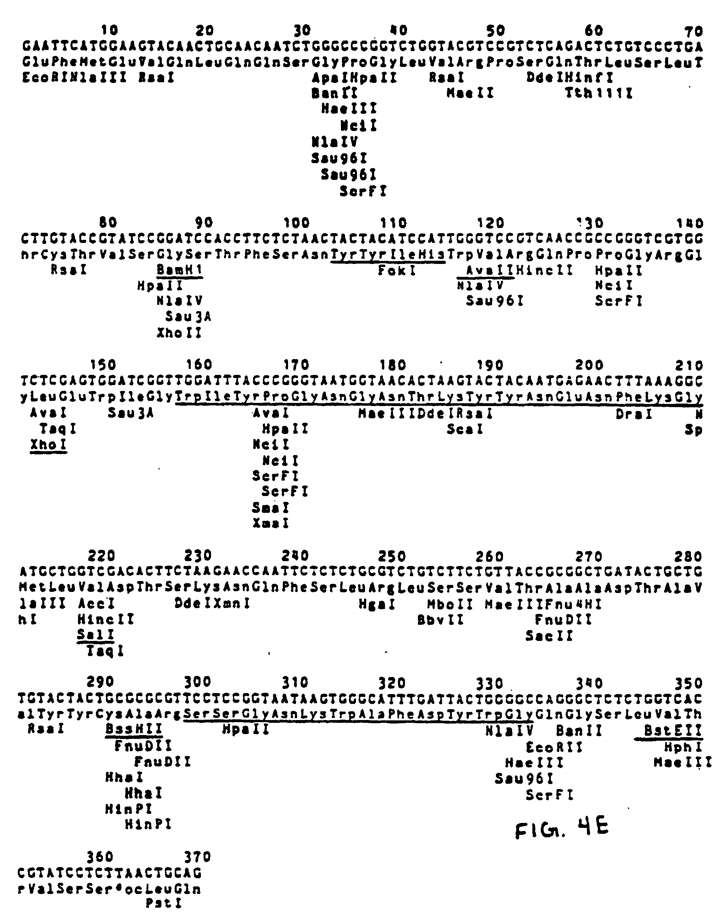

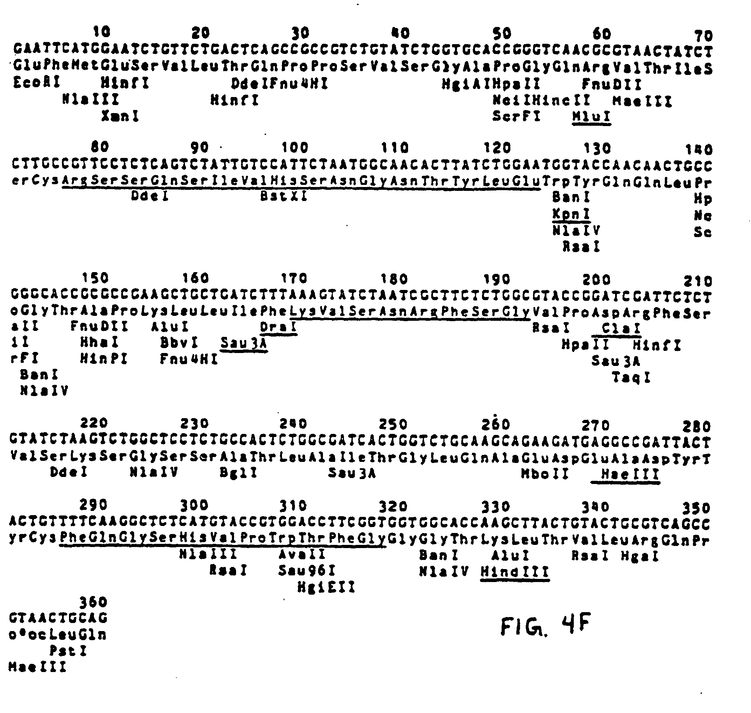

FRs from the heavy and light chain murine anti-digoxin monoclonal 26-10 (Figures 4A and 4B) were encoded on the same DNAs with CDRs from the murine anti-lysozyme monoclonal glp-4 heavy chain (Figure 3 sequence 1) and light chain to produce V_{H} (Figure 4C) and V_{L} (Figure 4D) regions together defining a biosynthetic antibody binding site which is specific for lysozyme. Murine CDRs from both the heavy and light chains of monoclonal glp-4 were encoded on the same DNAs with FRs from the heavy and light chains of human myeloma antibody NEWM (Figures 4E and 4F). The resulting interspecies chimeric antibody binding domain has reduced immunogenicity in humans because of its human FRs, and specificity for lysozyme because of its murine CDRs.

A synthetic DNA was designed to facilitate CDR insertions into a human heavy chain FR and to facilitate empirical refinement of the resulting chimeric amino acid sequence. This DNA is depicted in Figure 5.

A synthetic, bifunctional FB-binding site protein was also designed at the DNA level, expressed, purified, renatured, and shown to bind specifically with a preselected antigen (digoxin) and Fc. The detailed primary structure of this construct is shown in Figure 6; its tertiary structure is illustrated schematically in Figure 2B.

Details of these and other experiments, and additional design principles on which the invention is based, are set forth below.

### GENE DESIGN AND EXPRESSION

Given known variable region DNA sequences, synthetic V_{L} and V_{H} genes may be designed which encode native or near native FR and CDR amino acid sequences from an antibody molecule, each separated by unique restriction sites located as close to FR-CDR and CDR-FR borders as possible. Alternatively, genes may be designed which encode native FR sequences which are similar or identical to the FRs of an antibody molecule from a selected species, each separated by "dummy" CDR sequences containing strategically located restriction sites. These DNAs serve as starting materials for producing BABS, as the native or "dummy" CDR sequences may be excised and replaced with sequences encoding the CDR amino acids defining a selected binding site. Alternatively, one may design and directly synthesize native or near-native FR sequences from a first antibody molecule, and CDR sequences from a second antibody molecule. Any one of the V_{H} and V_{L} sequences described above may be linked together directly, via an amino acids chain or linker connecting the C-terminus of one chain with the N-terminus of the other.

These genes, once synthesized, may be cloned with or without additional DNA sequences coding for, e.g., an antibody constant region, enzyme, or toxin, or a leader peptide which facilitates secretion or intracellular stability of a fusion polypeptide. The genes then can be expressed directly in an appropriate host cell, or can be further engineered before expression by the exchange of FR, CDR, or "dummy" CDR sequences with new sequences. This . manipulation is facilitated by the presence of the restriction sites which have been engineered into the gene at the FR-CDR and CDR-FR borders.

Figure 3 illustrates the general approach to designing a chimeric V_{H}; further details of exemplary designs at the DNA level are shown in Figures 4A-4F. Figure 3, lines 1 and 2, show the amino acid sequences of the heavy chain variable region of the murine monoclonals glp-4 (anti-lysozyme) and 26-10 (anti-digoxin), including the four FR and three CDR sequences of each. Line 3 shows the sequence of a chimeric V_{H} which comprises 26-10 FRs and glp-4 CDRs. As illustrated, the hybrid protein of line 3 is identical to the native protein of line 2, except that 1) the sequence TFTNYYIHWLK has replaced the sequence IFTDFYMNWVR, 2) EWIGWIYPGNGNTKYNENFKG has replaced DYIGYISPYSGVTGYNQKFKG, 3) RYTHYYF has replaced GSSGNKWAM, and 4) A has replaced V as the sixth amino acid beyond CDR-2. These changes have the effect of changing the specificity of the 26-10 V_{H} to mimic the specificity of glp-4. The Ala to Val single amino acid replacement within the relatively conserved framework region of 26-10 is an example of the replacement of an amino acid outside the hypervariable region made for the purpose of altering specificity by CDR replacement. Beneath sequence 3 of Figure 3, the restriction sites in the DNA encoding the chimeric V_{H} (see Figures 4A-4F) are shown which are disposed about the CDR-FR borders.

Lines 4 and 5 of Figure 3 represent another construct. Line 4 is the full length V_{H} of the human antibody NEWM. That human antibody may be made specific for lysozyme by CDR replacement as shown in line 5. Thus, for example, the segment TFTNYYIHWLK from glp-4 replaces TFSNDYYTWVR of NEWM, and its other CDRs are replaced as shown. This results in a V_{H} comprising a human framework with murine sequences determining specificity.

By sequencing any antibody, or obtaining the sequence from the literature, in view of this disclosure one skilled in the art can produce a BABS of any desired specificity comprising any desired framework region. Diagrams such as Figure 3 comparing the amino acid sequence are valuable in suggesting which particular amino acids should be replaced to determine the desired complementarity. Expressed sequences may be tested for binding and refined by exchanging selected amino acids in relatively conserved regions, based on observation of trends in amino acid sequence data and/or computer modeling techniques.

Significant flexibility in V_{H} and V_{L} design is possible because the amino acid sequences are determined at the DNA level, and the manipulation of DNA can be accomplished easily.

For example, the DNA sequence for murine V_{H} and V_{L} 26-10 containing specific restriction sites flanking each of the three CDRs was designed with the aid of a commercially available computer program which performs combined reverse translation and restriction site searches ("RV.exe" by Compugene, Inc.). The known amino acid sequences for V_{H} and V_{L} 26-10 polypeptides were entered, and all potential DNA sequences which encode those peptides and all potential restriction sites were analyzed by the program. The program can, in addition, select DNA sequences encoding the peptide using only codons preferred by E. coli if this bacterium is to be host expression organism of choice. Figures 4A and 4B show an example of program output. The nucelic acid sequences of the synthetic gene and the corresponding amino acids are shown. Sites of restriction endonuclease cleavage are also indicated. The CDRs of these synthetic genes are underlined.

The DNA sequences for the synthetic 26-10 V_{H} and V_{L} are designed so that one or both of the restriction sites flanking each of the three CDRs are unique. A six base site (such as that recognized by Bsm I or BspM I) is preferred, but where six base sites are not possible, four or five base sites are used. These sites, if not already unique, are rendered unique within the gene by eliminating other occurrences within the gene without altering necessary amino acid sequences. Preferred cleavage sites are those that, once cleaved, yield fragments with sticky ends just outside of the boundary of the CDR within the framework. However, such ideal sites are only occasionally possible because the FR-CDR boundary is not an absolute one, and because the amino acid sequence of the FR may not permit a restriction site. In these cases, flanking sites in the FR which are more distant from the predicted boundary are selected.

Figure 5 discloses the nucleotide and corresponding amino acid sequence (shown in standard single letter code) of a synthetic DNA comprising a master framework gene having the generic structure:

R₁-FR₁-X₁-FR₂-X₂-FR₃-X₃-FR₄-R₂

where R₁ and R₂ are restricted ends which are to be ligated into a vector, and X₁, X₂, and X₃ are DNA sequences whose function is to provide convenient restriction sites for CDR insertion. This particular DNA has murine FR sequences and unique, 6-base restriction sites adjacent the FR borders so that nucleotide sequences encoding CDRs from a desired monoclonal can be inserted easily. Restriction endonuclease digestion sites are indicated with their abbreviations; enzymes of choice for CDR replacement are underscored. Digestion of the gene with the following restriction endonucleases results in 3' and 5' ends which can easily be matched up with and ligated to native or synthetic CDRs of desired specificity; KpnI and BstXI are used for ligation of CDR₁; XbaI and Dral for CDR₂; and BssHII and ClaI for CDR₃.

### OLIGONUCLEOTIDE SYNTHESIS

The synthetic genes and DNA fragments designed as described above preferably are produced by assembly of chemically synthesized oligonucleotides. 15-100mer oligonucleotides may be synthesized on a Biosearch DNA Model 8600 Synthesizer, and purified by polyacrylamide gel electrophoresis (PAGE) in Tris-Borate-EDTA buffer (TBE). The DNA is then electroeluted from the gel. Overlapping oligomers may be phosphorylated by T4 polynucleotide kinase and ligated into larger blocks which may also be purified by PAGE.

### CLONING OF SYNTHETIC OLIGONUCLEOTIDES

The blocks or the pairs of longer oligonucleotides may be cloned into E. coli using a suitable, e.g., pUC, cloning vector. Initially, this vector may be altered by single strand mutagenesis to eliminate residual six base altered sites. For example, V_{H} may be synthesized and cloned into pUC as five primary blocks spanning the following restriction sites: 1. EcoRI to first NarI site; 2. first NarI to XbaI; 3. XbaI to SalI; 4. Sall to NcoI; 5. NcoI to BamHI. These cloned fragments may then be isolated and assembled in several three-fragment ligations and cloning steps into the pUC8 plasmid. Desired ligations selected by PAGE are then transformed into, for example, E. coli strain JM83, and plated onto LB Ampicillin + Xgal plates according to standard procedures. The gene sequence may be confirmed by supercoil sequencing after cloning, or after subcloning into M13 via the dideoxy method of Sanger.

### PRINCIPLE OF CDR EXCHANGE

Three CDRs (or alternatively, four FRs) can be replaced per V_{H} or V_{L}. In simple cases, this can be accomplished by cutting the shuttle pUC plasmid containing the respective genes at the two unique restriction sites flanking each CDR or FR, removing the excised sequence, and ligating the vector with a native nucleic acid sequence or a synthetic oligonucleotide encoding the desired CDR or FR. This three part procedure would have to be repeated three times for total CDR replacement and four times for total FR replacement. Alternatively, a synthetic nucleotide encoding two consecutive CDRs separated by the appropriate FR can be ligated to a pUC or other plasmid containing a gene whose corresponding CDRs and FR have been cleaved out. This procedure reduces the number of steps required to perform CDR and/or FR exchange.

### EXPRESSION OF PROTEINS

The engineered genes can be expressed in appropriate prokaryotic hosts such as various strains of E. coli, and in eucaryotic hosts such as Chinese hamster ovary cell, murine myeloma, and human myeloma/transfectoma cells.

For example, if the gene is to be expressed in E. coli, it may first be cloned into an expression vector. This is accomplished by positioning the engineered gene downstream from a promoter sequence such as trp or tac, and a gene coding for a leader peptide. The resulting expressed fusion protein accumulates in refractile bodies in the cytoplasm of the cells, and may be harvested after disruption of the cells by French press or sonication. The refractile bodies are solubilized, and the expressed proteins refolded and cleaved by the methods already established for many other recombinant proteins.

If the engineered gene is to be expressed in myeloma cells, the conventional expression system for immunoglobulins, it is first inserted into an expression vector containing, for example, the Ig promoter, a secretion signal, immunoglobulin enhancers, and various introns. This plasmid may also contain sequences encoding all or part of a constant region, enabling an entire part of a heavy or light chain to be expressed. The gene is transfected into myeloma cells via established electroporation or protoplast fusion methods. Cells so transfected can express V_{L} or V_{H} fragments, V_{L2} or V_{H2} homodimers, V_{L}-V_{H} heterodimers, V_{H}-V_{L} or V_{L}-V_{H} single chain polypeptides, complete heavy or light immunoglobulin chains, or portions thereof, each of which may be attached in the various ways discussed above to a protein region having another function (e.g., cytotoxicity).

Vectors containing a heavy chain V region (or V and C regions) can be cotransfected with analogous vectors carrying a light chain V region (or V and C regions), allowing for the expression of noncovalently associated binding sites (or complete antibody molecules).

In the examples which follow, a specific example of how to make a single chain binding site is disclosed, together with methods employed to assess its binding properties. Thereafter, a protein construct having two functional domains is disclosed. Lastly, there is disclosed a series of additional targeted proteins.

### I EXAMPLE OF CDR EXCHANGE AND EXPRESSION

The synthetic gene coding for murine V_{H} and V_{L} 26-10 shown in Figures 4A and 4B were designed from the known amino acid sequence of the protein with the aid of Compugene, a software program. These genes, although coding for the native amino acid sequences, also contain non-native and often unique restriction sites flanking nucleic acid sequences encoding CDR's to facilitate CDR replacement as noted above.

Both the 3' and 5' ends of the large synthetic oligomers were designed to include 6-base restriction sites, present in the genes and the pUC vector. Furthermore, those restriction sites in the synthetic genes which were only suited for assembly but not for cloning the pUC were extended by "helper" cloning sites with matching sites in pUC.

Cloning of the synthetic DNA and later assembly of the gene is facilitated by the spacing of unique restriction sites along the gene. This allows corrections and modifications by cassette mutagenesis at any location. Among them are alterations near the 5' or 3' ends of the gene as needed for the adaptation to different expression vectors. For example, a PstI site is positioned near the 5' end of the V_{H} gene. Synthetic linkers can be attached easily between this site and a restriction site in the expression plasmid. These genes were synthesized by assembling oligonucleotides as described above using a Biosearch Model 8600 DNA Synthesizer. They were ligated to vector pUC8 for transformation of E. coli.

Specific CDRs may be cleaved from the synthetic V_{H} gene by digestion with the following pairs of restriction endonucleases: HpHI and BstXI for CDR₁; XbaI and DraI for CDR₂; and BanII and BanI for CDR₃. After removal on one CDR, another CDR of desired specificity may be ligated directly into the restricted gene, in its place if the 3' and 5' ends of the restricted gene and the new CDR contain complementary single stranded DNA sequences.

In the present example, the three CDRs of each of murine V_{H} 26-10 and V_{L} 26-10 were replaced with the corresponding CDRs of glp-4. The nucleic acid sequences and corresponding amino acid sequences of the chimeric V_{H} and V_{L} genes encoding the FRs of 26-10 and CDRs of glp-4 are shown in Figures 4C and 4D. The positions of the restriction endonuclease cleavage sites are noted with their standard abbreviations. CDR sequences are underlined as are the restriction endonucleases of choice useful for further CDR replacement.

These genes were cloned into pUC8, a shuttle plasmid. To retain unique restriction sites after cloning, the V_{H}-like gene was spliced into the EcoRl and HindIII or BamHI sites of the plasmid.

Direct expression of the genes may be achieved in E. coli. Alternatively, the gene may be preceded by a leader sequence and expressed in E. coli as a fusion product by splicing the fusion gene into the host gene whose expression is regulated by interaction of a repressor with the respective operator. The protein can be induced by starvation in minimal medium and by chemical inducers. The V_{H}-V_{L} biosynthetic 26-10 gene has been expressed as such a fusion protein behind the trp and tac promoters. The gene translation product of interest may then be cleaved from the leader in the fusion protein by e.g., cyanogen bromide degradation, tryptic digestion, mild acid cleavage, and/or digestion with factor Xa protease. Therefore, a shuttle plasmid containing a synthetic gene encoding a leader peptide having a site for mild acid cleavage, and into which has been spliced the synthetic BABS gene was used for this purpose. In addition, synthetic DNA sequences encoding a signal peptide for secretion of the processed target protein into the periplasm of the host cell can also be incorporated into the plasmid.

After harvesting the gene product and optionally releasing it from a fusion peptide, its activity as an antibody binding site and its specificity for glp-4 (lysozyme) epitope are assayed by established immunological techniques, e.g., affinity chromatography and radioimmunoassay. Correct folding of the protein to yield the proper three-dimensional conformation of the antibody binding site is prerequisite for its activity. This occurs spontaneously in a host such as a myeloma cell which naturally expresses immunoglobulin proteins. Alternatively, for bacterial expression, the protein forms inclusion bodies which, after harvesting, must be subjected to a specific sequence of solvent conditions (e.g., diluted 20 X from 8 M urea 0.1 M Tris-HCl pH 9 into 0.15 M NaCl, 0.01 M sodium phosphate, pH 7.4 (Hochman et al. (1976) Biochem. 15:2706-2710) to assume its correct conformation and hence its active form.

Figures 4E and 4F show the DNA and amino acid sequence of chimeric V_{H} and V_{L} comprising human FRs from NEWM and murine CDRs from glp-4. The CDRs are underlined, as are restriction sites of choice for further CDR replacement or empirically determined refinement.

These constructs also constitute master framework genes, this time constructed of human framework sequences. They may be used to construct BABS of any desired specificity by appropriate CDR replacement.

Binding sites with other specificities have also been designed using the methodologies disclosed herein. Examples include those having FRs from the human NEWM antibody and CDRs from murine 26-10 (Figure 9A), murine 26-10 FRs and G-loop CDRs (Figure 9B), FRs and CDRs from murine MOPC-315 (Figure 9C), FRs and CDRs from an anti-human carcinoembryonic antigen monoclonal antibody (Figure 9D), and FRs and CDRs 1, 2, and 3 from V_{L} and FRs and CDR 1 and 3 from the V_{H} of the anti-CEA antibody, with CDR 2 from a consensus immunoglobulin gene (Figure 9E).

### II. Model Binding Site:

The digoxin binding site of the IgG_{2a,k} monoclonal antibody 26-10 has been analyzed by Mudgett-Hunter and colleagues (unpublished). The 26-10 V region sequences were determined from both amino acid sequencing and DNA sequencing of 26-10 H and L chain mRNA transcripts (D. Panka, J.N. & M.N.M., unpublished data). The 26-10 antibody exhibits a high digoxin binding affinity [Kₒ = 5.4 X 10⁹ M⁻¹] and has a well-defined specificity profile, providing a baseline for comparison with the biosynthetic binding sites mimicking its structure.

### Protein Design:

Crystallographically determined atomic coordinates for Fab fragments of 26-10 were obtained from the Brookhaven Data Bank. Inspection of the available three-dimensional structures of Fv regions within their parent Fab fragments indicated that the Euclidean distance between the C-terminus of the V_{H} domain and the N-terminus of the V_{L} domain is about 3.5 nm (35 A). Considering that the peptide unit length is approximately 3.8 A, a 15 residue linker was selected to bridge this gap. The linker was designed so as to exhibit little propensity for secondary structure and not to interfere with domain folding. Thus, the 15 residue sequence (Gly-Gly-Gly-Gly-Ser)₃ was selected to connect the V_{H} carboxyl- and V_{L} amino-termini.

Binding studies with single chain binding sites having less than or greater than 15 residues demonstrate the importance of the prerequisite distance which must separate V_{H} from V_{L}; for example, a (Gly₄-Ser)₁ linker does not demonstrate binding activity, and those with (Gly₄-Ser)₅ linkers exhibit very low activity compared to those with (Gly₄-Ser)₃ linkers.

### Gene Synthesis:

Design of the 744 base sequence for the synthetic binding site gene was derived from the Fv protein sequence of 26-10 by choosing codons frequently used in E. coli. The model of this representative synthetic gene is shown in Figure 8, discussed previously. Synthetic genes coding for the trp promoter-operator, the modified trp LE leader peptide (MLE), the sequence of which is shown in Figure 10A, and V_{H} were prepared largely as described previously. The gene coding for V_{H} was assembled from 46 chemically synthesized oligonucleotides, all 15 bases long, except for terminal fragments (13 to 19 bases) that included cohesive cloning ends. Between 8 and 15 overlapping oligonucleotides were enzymatically ligated into double stranded DNA, cut at restriction sites suitable for cloning (Narl, XbaI, SalI, SacII, SacI), purified by PAGE on 8% gels, and cloned in pUC which was modified to contain additional cloning sites in the polylinker. The cloned segments were assembled stepwise into the complete gene mimicking V_{H} by ligations in the pUC cloning vector.

The gene mimicking 26-10 V_{L} was assembled from 12 long synthetic polynucleotides ranging in size from 33 to 88 base pairs, prepared in automated DNA synthesizers (Model 6500, Biosearch, San Rafael, CA; Model 380A, Applied Biosystems, Foster City, CA). Five individual double stranded segments were made out of pairs of long synthetic oligonucleotides spanning six-base restriction sites in the gene (AatII, BstEII, PpnI, HindIII, BglII, and PstI). In one case, four long overlapping strands were combined and cloned. Gene fragments bounded by restriction sites for assembly that were absent from the pUC polylinker, such as AatII and BstEII, were flanked by EcoRI and BamHI ends to facilitate cloning.

The linker between V_{H} and V_{L}, encoding (Gly-Gly-Gly-Gly-Ser)₃, was cloned from two long synthetic oligonucleotides, 54 and 62 bases long, spanning SacI and AatII sites, the latter followed by an EcoRI cloning end. The complete single chain binding site gene was assembled from the V_{H}, V_{L}, and linker genes to produce a construct, corresponding to aspartyl-prolyl-V_{H}-(linker)-V_{L}, flanked by EcoRI and PstI restriction sites.

The trp promoter-operator, starting from its SspI site, was assembled from 12 overlapping 15 base oligomers, and the MLE leader gene was assembled from 24 overlapping 15 base oligomers. These were cloned and assembled in pUC using the strategy of assembly sites flanked by cloning sites. The final expression plasmid was constructed in the pBR322 vector by a 3-part ligation using the sites SspI, EcoRI, and PstI (see Figure 10B). Intermediate DNA fragments and assembled genes were sequenced by the dideoxy method.

### Fusion Protein Expression:

Single-chain protein was expressed as a fusion protein. The MLE leader gene (Fig. 10A) was derived from E. coli trp LE sequence and expressed under the control of a synthetic trp promoter and operator. E. coli strain JM83 was transformed with the expression plasmid and protein expression was induced in M9 minimal medium by addition of indoleacrylic acid (10 µg/ml) at a cell density with A₆₀₀ = 1. The high expression levels of the fusion protein resulted in its accumulation as insoluble protein granules, which were harvested from cell paste (Figure 11, Lane 1).

### Fusion Protein Cleavage:

The MLE leader was removed from the binding site protein by acid cleavage of the Asp-Pro peptide bond engineered at the junction of the MLE and binding site sequences. The washed protein granules containing the fusion protein were cleaved in 6 M guanidine-HCl + 10% acetic acid, pH 2.5, incubated at -37°C for 96 hrs. The reaction was stopped through precipitation by addition of a 10-fold excess of ethanol with overnight incubation at -20°C, followed by centrifugation and storage at -20°C until further purification (Figure 11, Lane 2).

### Protein Purification:

The acid cleaved binding site was separated from remaining intact fused protein species by chromatography on DEAE cellulose. The precipitate obtained from the cleavage mixture was redissolved in 6 M guanidine-HCl + 0.2 M Tris-HCl, pH 8.2, + 0.1 M 2-mercaptoethanol and dialyzed exhaustively against 6 M urea + 2.5 mM Tris-HCl, pH 7.5, + 1 mM EDTA. 2-Mercaptoethanol was added to a final concentration of 0.1 M, the solution was incubated for 2 hrs at room temperature and loaded onto a 2.5 X 45 cm column of DEAE cellulose (Whatman DE 52), equilibrated with 6 M urea + 2.5 mM Tris-HCl + 1 mM EDTA, pH 7.5. The intact fusion protein bound weakly to the DE 52 column such that its elution was retarded relative to that of the binding protein. The first protein fractions which eluted from the column after loading and washing with urea buffer contained BABS protein devoid of intact fusion protein. Later fractions contaminated with some fused protein were pooled, rechromatographed on DE 52, and recovered single chain binding protein combined with other purified protein into a single pool (Figure 11, Lane 3).

### Refolding:

The 26-10 binding site mimic was refolded as follows: the DE 52 pool, disposed in 6 M urea + 2.5 mM Tris-HCl + 1 mM EDTA, was adjusted to pH 8 and reduced with 0.1 M 2-mercaptoethanol at 37°C for 90 min. This was diluted at least 100-fold with 0.01 M sodium acetate, pH 5.5, to a concentration below 10 µg/ml and dialyzed at 4°C for 2 days against acetate buffer.

### Affinity Chromatography:

Purification of active binding protein by affinity chromatography at 4°C on a ouabain-amine-Sepharose column was performed. The dilute solution of refolded protein was loaded directly onto a pair of tandem columns, each containing 3 ml of resin equilibrated with the 0.01 M acetate buffer, pH 5.5. The columns were washed individually with an excess of the acetate buffer, and then by sequential additions of 5 ml each of 1 M NaCl, 20 mM ouabain, and 3 M potassium thiocyanate dissolved in the acetate buffer, interspersed with acetate buffer washes. Since digoxin binding activity was still present in the eluate, the eluate was pooled and concentrated 20-fold by ultrafiltration (PM 10 membrane, 200 ml concentrator; Amicon), reapplied to the affinity columns, and eluted as described. Fractions with significant absorbance at 280 nm were pooled and dialyzed against PBSA or the above acetate buffer. The amounts of protein in the DE 52 and ouabain-Sepharose pools were quantitated by amino acid analysis following dialysis against 0.01 M acetate buffer. The results are shown below in Table 1.

**TABLE 1**

| Estimated Yields of BABS Protein During Purification | | | | |
|---|---|---|---|---|
| Step | Wet wt. Per 1 | mg protein | Cleavage yield (%) prior step | Yield relative to fusion protein |
| Cell paste | 12.0 g | 1440.0 mg^{a} | | |
| Fusion protein Granules | 2.3 g | 480.0 mg^{a}^{,}^{b} | 100.0% | 100.0% |
| Acid Cleavage/DE 52 pool | | 144.0 mg | 38.0^{e} | 38.0^{e} |
| Ouabain-Sepharose pool | | 18.1 mg | 12.6^{d} | 4.7^{e} |

| | | | | |
|---|---|---|---|---|
| ^{a}Determined by Lowry protein analysis | | | | |
| ^{b}Determined by absorbance measurements | | | | |
| ^{c}Determined by amino acid analysis ^{d}Calculated from the amount of BABS protein specifically eluted from ouabain-Sepharose relative to that applied to the resin; values were determined by amino acid analysis | | | | |
| ^{e}Percentage yield calculated on a molar basis | | | | |

### Sequence Analysis of Gene and Protein:

The complete gene was sequenced in both directions using the dideoxy method of Sanger which confirmed the gene was correctly assembled. The protein sequence was also verified by protein sequencing. Automated Edman degradation was conducted on intact protein (residues 1-40), as well as on two major CNBr fragments (residues 108-129 and 140-159) with a Model 470A gas phase sequencer equipped with a Model 120A on-line phenylthiohydantoin-amino acid analyzer (Applied Biosystems, Foster City, CA). Homogeneous binding protein fractionated by SDS-PAGE and eluted from gel strips with water, was treated with a 20,000-fold excess of CNBr, in 1% trifluoroacetic acid-acetonitrile (1:1), for 12 hrs at 25° (in the dark). The resulting fragments were separated by SDS-PAGE and transferred electrophoretically onto an Immobilon membrane (Millipore, Bedford, MA), from which stained bands were cut out and sequenced.

### Specificity Determination:

Specificities of anti-digoxin 26-10 Fab and the BABS were assessed by radioimmunoassay. Wells of microtiter plates were coated with affinity-purified goat anti-murine Fab fragment (ICN ImmunoBiologicals, Lisle, IL) at 10 µg/ml in PBSA overnight at 4°C. After the plates were washed and blocked with 1% horse serum in PBSA, solutions (50 µl) containing 26-10 Fab or the BABS in either PBSA or 0.01 M sodium acetate at pH 5.5 were added to the wells and incubated 2-3 hrs at room temperature. After unbound antibody fragment was washed from the wells, 25 µl of a series of concentrations of cardiac glycosides (10⁻⁴ to 10⁻¹¹ M in PBSA) were added. The cardiac glycosides tested included digoxin, digitoxin, digoxigenin, digitoxigenin, gitoxin, ouabain, and acetyl strophanthidin. After the addition of ¹²⁵I-digoxin (25 µl, 50,000 cpm; Cambridge Diagnostics, Billerica, MA) to each well, the plates were incubated overnight at 4°C, washed and counted. The inhibition curves are plotted in Figure 12. The relative affinities for each digoxin analogue were calculated by dividing the concentration of each analogue at 50% inhibition by the concentration of digoxin (or digoxigenin) that gave 50% inhibition. There is a displacement of inhibition curves for the BABS to lower glycoside concentrations than observed for 26-10 Fab, because less active BABS than 26-10 Fab was bound to the plate. When 0.25 M urea was added to the BABS in 0.01 M sodium acetate, pH 5.5, more active sFv was bound to the goat anti-murine Fab coating on the plate. This caused the BABS inhibition curves to shift toward higher glycoside concentrations, closer to the position of those for 26-10 Fab, although maintaining the relative positions of curves for sFv obtained in acetate buffer alone. The results, expressed as normalized concentration of inhibitor giving 50% inhibition of ¹²⁵I-digoxin binding, are shown in Table 2.

**TABLE 2**

| 26-10 Antibody Species | Normalizing Glycoside | D | DG | DO | DOG | A-S | G | O |
|---|---|---|---|---|---|---|---|---|
| Fab | Digoxin | 1.0 | 1.2 | 0.9 | 1.0 | 1.3 | 9.6 | 15 |
| | Digoxigenin | 0.9 | 1.0 | 0.8 | 0.9 | 1.1 | 8.1 | 13 |
| BABS | Digoxin | 1.0 | 7.3 | 2.0 | 2.6 | 5.9 | 62 | 150 |
| | Digoxigenin | 0.1 | 1.0 | 0.3 | 0.4 | 0.8 | 8.5 | 21 |
| D = Digoxin | | | | | | | | |
| DG = Digoxigenin | | | | | | | | |
| DO = Digitoxin | | | | | | | | |
| DOG = Digitoxigenin | | | | | | | | |
| A-S = Acetyl Strophanthidin | | | | | | | | |
| G = Gitoxin | | | | | | | | |
| O = Ouabain | | | | | | | | |

### Affinity Determination:

Association constants were measured by equilibrium binding studies. In immunoprecipitation experiments, 100 µl of ³H-digoxin (New England Nuclear, Billerica, MA) at a series of concentrations (10⁻⁷ M to 10⁻¹¹ M) were added to 100 µl of 26-10 Fab or the BABS at a fixed concentration. After 2-3 hrs of incubation at room temperature, the protein was precipitated by the addition of 100 µl goat antiserum to murine Fab fragment (ICN ImmunoBiologicals), 50 µl of the IgG fraction of rabbit anti-goat IgG (ICN ImmunoBiologicals), and 50 µl of a 10% suspension of protein A-Sepharose (Sigma). Following 2 hrs at 4°C, bound and free antigen were separated by vacuum filtration on glass fiber filters (Vacuum Filtration Manifold, Millipore, Bedford, MA). Filter disks were then counted in 5 ml of scintillation fluid with a Model 1500 Tri-Carb Liquid Scintillation Analyzer (Packard, Sterling, VA). The association constants, Kₒ, were calculated from Scatchard analyses of the untransformed radioligand binding data using LIGAND, a non-linear curve fitting program based on mass action. Kₒs were also calculated by Sips plots and binding isotherms shown in Figure 13A for the BABS and 13B for the Fab. For binding isotherms, data are plotted as the concentration of digoxin bound versus the log of the unbound digoxin concentration, and the dissociation constant is estimated from the ligand concentration at 50% saturation. These binding data are also plotted in linear form as Sips plots (inset), having the same abscissa as the binding isotherm but with the ordinate representing log r/(n-r), defined below. The average intrinsic association constant (Kₒ) was calculated from the modified Sips equation (39), log (r/n-r) = a log C - a log Kₒ, where r equals moles of digoxin bound per mole of antibody at an unbound digoxin concentration equal to C; n is the number of moles of digoxin bound at saturation of the antibody binding site, and a is an index of heterogeneity which describes the distribution of association constants about the average intrinsic association constant Kₒ. Least squares linear regression analysis of the data indicated correlation coefficients for the lines obtained were 0.96 for the BABS and 0.99 for 26-10 Fab. A summary of the calculated association constants are shown below in Table 3.

**TABLE 3**

| Method of Data Analysis | Association Constant, Kₒ | |
|---|---|---|
| | Kₒ (BABS), M⁻¹ | Kₒ (Fab), M⁻¹ |
| Scatchard plot | (3.2 ± 0.9) X 10⁷ | (1.9 ± 0.2) X 10⁸ |
| Sips plot | 2.6 X 10⁷ | 1.8 X 10⁸ |
| Binding isotherm | 5.2 X 10⁷ | 3.3 X 10⁸ |

### III. Synthesis of a Multifunctional Protein

A nucleic acid sequence encoding the single chain binding site described above was fused with a sequence encoding the FB fragment of protein A as a leader to function as a second active region. As a spacer, the native amino acids comprising the last 11 amino acids of the FB fragment bonded to an Asp-Pro dilute acid cleavage site was employed. The FB binding domain of the FB consists of the immediately preceding 43 amino acids which assume a helical configuration (see Fig. 2B).

The gene fragments are synthesized using a Biosearch DNA Model 8600 Synthesizer as described above. Synthetic oligonucleotides are cloned according to established protocol described above using the pUC8 vector transfected into E. coli. The completed fused gene set forth in Figure 6A is then expressed in E. coli.

After sonication, inclusion bodies were collected by centrifugation, and dissolved in 6 M guanidine hydrochloride (GuHCl), 0.2 M Tris, and 0.1 M 2-mercaptoethanol (BME), pH 8.2. The protein was denatured and reduced in the solvent overnight at room temperature. Size exclusion chromatography was used to purify fusion protein from the inclusion bodies. A Sepharose 4B column (1.5 X 80 cm) was run in a solvent of 6 M GuHCl and 0.01 M NaOAc, pH 4.75. The protein solution was applied to the column at room temperature in 0.5-1.0 ml amounts. Fractions were collected and precipitated with cold ethanol. These were run on SDS gels, and fractions rich in the recombinant protein (approximately 34,000 D) were pooled. This offers a simple first step for cleaning up inclusion body preparations without suffering significant proteolytic degradation.

For refolding, the protein was dialyzed against 100 ml of the same GuHCl-Tris-BME solution, and dialysate was diluted 11-fold over two days to 0.55 M GuHCl, 0.01 M Tris, and 0.01 M BME. The dialysis sacks were then transferred to 0.01 M NaCl, and the protein was dialyzed exhaustively before being assayed by RIA's for binding of ¹²⁵I-labelled digoxin. The refolding procedure can be simplified by making a rapid dilution with water to reduce the GuHCl concentration to 1.1 M, and then dialyzing against phosphate buffered saline (0.15 M NaCl, 0.05 M potassium phosphate, pH 7, containing 0.03% NaN₃), so that it is free of any GuHCl within 12 hours. Product of both types of preparation showed binding activity, as indicated in Figure 7A.

### Demonstration of Bifunctionality:

This protein with an FB leader and a fused BABS is bifunctional; the BABS can bind the antigen and the FB can bind the Fc regions of immunoglobulins. To demonstrate this dual and simulataneous activity several radioimmunoassays were performed.

Properties of the binding site were probed by a modification of an assay developed by Mudgett-Hunter et al. (J. Immunol. (1982) 129:1165-1172; Molec. Immunol. (1985) 22:477-488), so that it could be run on microtiter plates as a solid phase sandwich assay. Binding data were collected using goat anti-murine Fab antisera (gAmFab) as the primary antibody that initially coats the wells of the plate. These are polyclonal antisera which recognize epitopes that appear to reside mostly on framework regions. The samples of interest are next added to the coated wells and incubated with the gAmFab, which binds species that exhibit appropriate antigenic sites. After washing away unbound protein, the wells are exposed to ¹²⁵I-labelled (radioiodinated) digoxin conjugates, either as ¹²⁵I-dig-BSA or ¹²⁵I-dig-lysine.

The data are plotted in Figure 7A, which shows the results of a dilution curve experiment in which the parent 26-10 antibody was included as a control. The sites were probed with ¹²⁵I-dig-BSA as described above, with a series of dilutions prepared from initial stock solutions, including both the slowly refolded (1) and fast diluted/quickly refolded (2) single chain proteins. The parallelism between all three dilution curves indicates that gAmFab binding regions on the BABS molecule are essentially the same as on the Fv of authentic 26-10 antibody, i.e., the surface epitopes appear to be the same for both proteins.

The sensitivity of these assays is such that binding affinity of the Fv for digoxin must be at least 10⁶. Experimental data on digoxin binding yielded binding constants in the range of 10⁸ to 10⁹ M⁻¹. The parent 26-10 antibody has an affinity of 5.4 X 10⁹ M⁻¹. Inhibition assays also indicate the binding of ¹²⁵I-dig-lysine, and can be inhibited by unlabelled digoxin, digoxigenin, digitoxin, digitoxigenin, gitoxin, acetyl strophanthidin, and ouabain in a way largely parallel to the parent 26-10 Fab. This indicates that the specificity of the biosynthetic protein is substantially identical to the original monoclonal.

In a second type of assay, Digoxin-BSA is used to coat microtiter plates. Renatured BABS (FB-BABS) is added to the coated plates so that only molecules that have a competent binding site can stick to the plate. ¹²⁵I-labelled rabbit IgG (radioligand) is mixed with bound FB-BABS on the plates. Bound radioactivity reflects the interation of IgG with the FB domain of the BABS, and the specificity of this binding is demonstrated by its inhibition with increasing amounts of FB, Protein A, rabbit IgG, IgG2a, and IgG1, as shown in Figure 7B.

The following species were tested in order to demonstrate authentic binding: unlabelled rabbit IgG and IgG2a monoclonal antibody (which binds competiviely to the FB domain of the BABS); and protein A and FB (which bind competively to the radioligand). As shown in Figure 7B, these species are found to completely inhibit radioligand binding, as expected. A monoclonal antibody of the IgG1 subclass binds poorly to the FB, as expected, inhibiting only about 34% of the radioligand from binding. These data indicate that the BABS domain and the FB domain have independent activity.

### IV. OTHER CONSTRUCTS

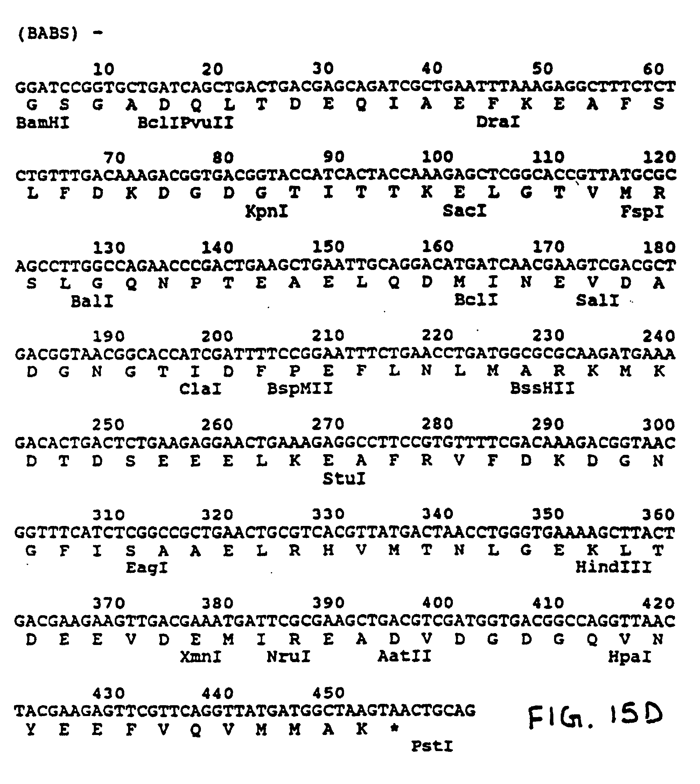

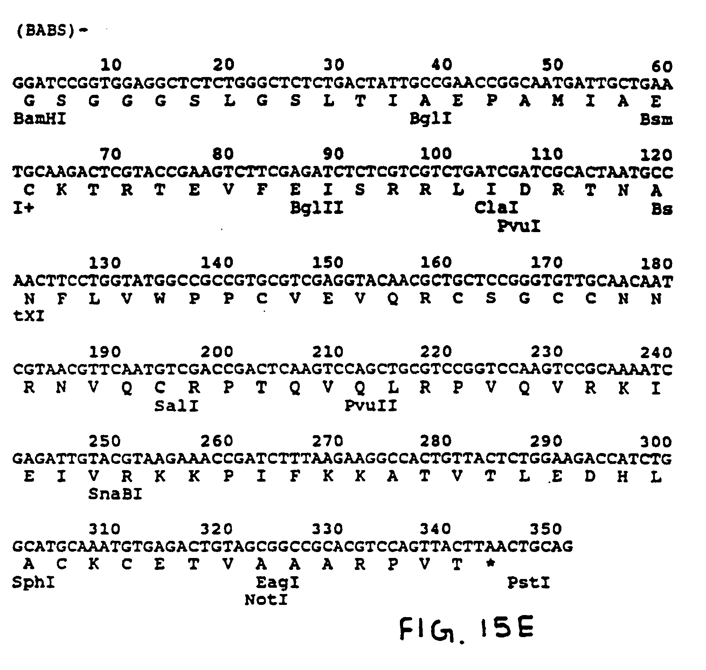

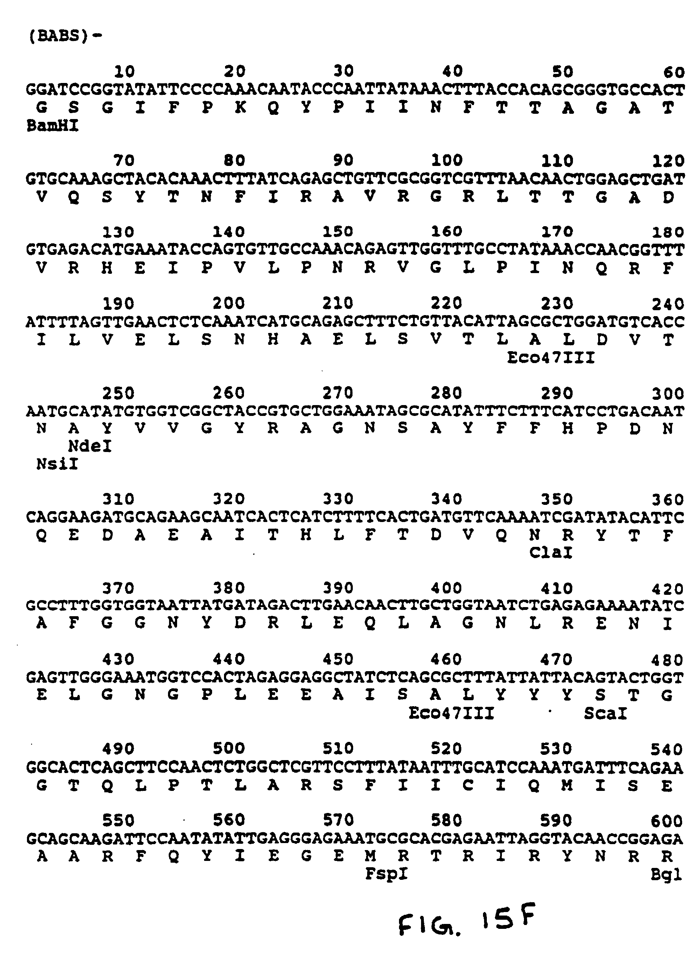

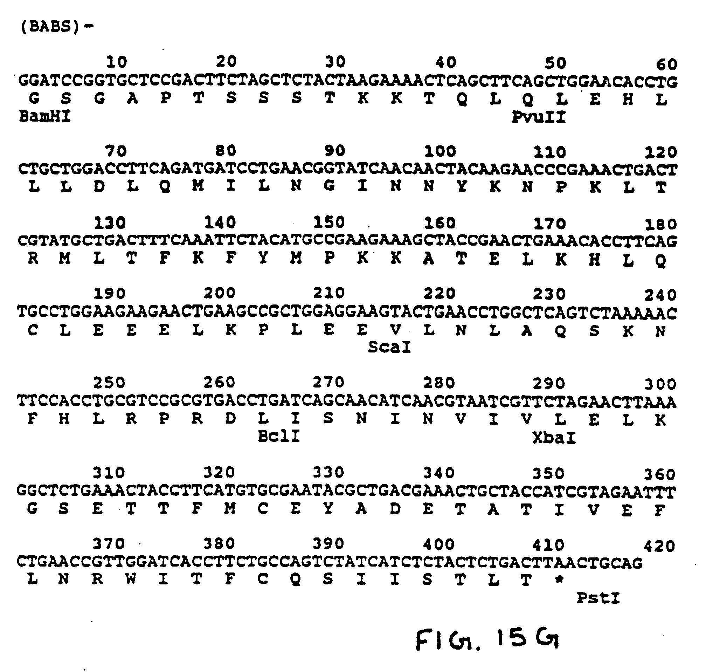

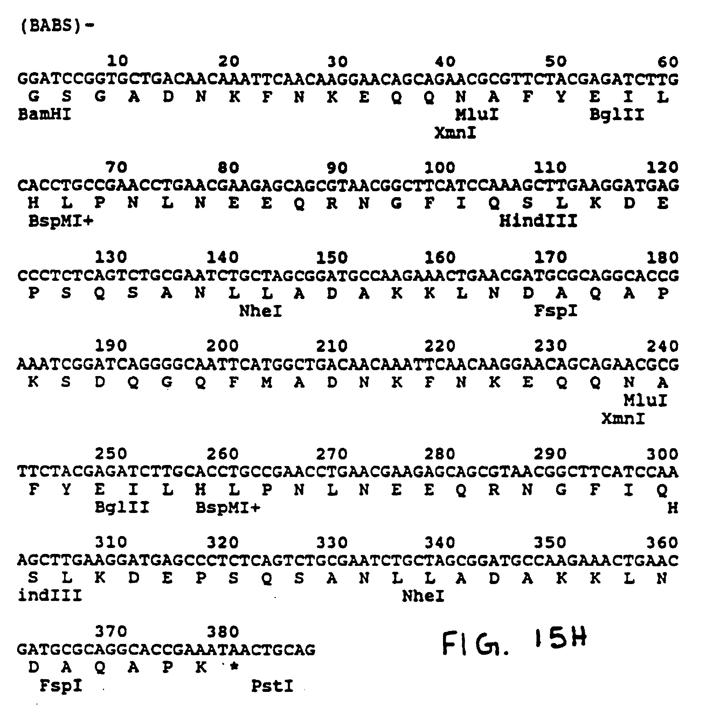

Other BASS-containing protein expressible in E. coli and other host cells as described above are set forth in the drawing. These proteins may be bifunctional or multifunctional. Each construct includes a single chain BABS linked via a spacer sequence to an effector molecule comprising amino acids encoding a biologically active effector protein such as an enzyme, receptor, toxin, or growth factor. Some examples of such constructs shown in the drawing include proteins comprising epidermal growth factor (EGF) (Figure 15A), streptavidin (Figure 15B), tumor necrosis factor (TNF) (Figure 15C), calmodulin (Figure 15D) the beta chain of platelet derived growth factor (B-PDGF) (15E) ricin A (15F), interleukin 2 (15G) and FB dimer (15H). Each is used as a trailer and is connected to a preselected BABS via a spacer (Gly-Ser-Gly) encoded by DNA defining a BamHI restriction site. Additional amino acids may be added to the spacer for empirical refinement of the construct if necessary by opening up the Bam HI site and inserting an oligonucleotide of a desired length having BamHI sticky ends. Each gene also terminates with a PstI site to facilitate insertion into a suitable expression vector.

The BABS of the EGF and PDGF constructs may be, for example, specific for fibrin so that the EGF or PDGF is delivered to the site of a wound. The BABS for TNF and ricin A may be specific to a tumor antigen, e.g., CEA, to produce a construct useful in cancer therapy. The calmodulin construct binds radioactive ions and other metal ions. Its BABS may be specific, for example, to fibrin or a tumor antigen, so that it can be used as an imaging agent to locate a thrombus or tumor. The streptavadin construct binds with biotin with very high affinity. The biotin may be labeled with a remotely detectable ion for imaging purposes. Alternatively, the biotin may be immobilized on an affinity matrix or solid support. The BABS-streptavidin protein could then be bound to the matrix or support for affinity chromatography or solid phase immunoassay. The interleukin-2 construct could be linked, for example, to a BABS specific for a T-cell surface antigen. The FB-FB dimer binds to Fc, and could be used with a BABS in an immunoassay or affinity purification procedure linked to a solid phase through immobilized immunoglobulin.

Figure 14 exemplifies a multifunctional protein having an effector segment as a leader. It comprises an FB-FB dimer linked through its C-terminal via an Asp-Pro dipeptide to a BABS of choice. It functions in a way very similar to the construct of Fig. 15H. The dimer binds avidly to the Fc portion of immunoglobulin. This type of construct can accordingly also be used in affinity chromatography, solid phase immunoassay, and in therapeutic contexts where coupling of immunoglobulins to another epitope is desired.

In view of the foregoing, it should be apparent that the invention is unlimited with respect to the specific types single polypeptide chains and linkers as well as the types of BABS and effector proteins to be linked. Accordingly, other embodiments are within the following claims.

Also contemplated is a biosynthetic binding protein expressed from DNA derived by recombinant techniques
said binding protein comprising a single polypeptide chain comprising at least two polypeptide domains connected by a polypeptide linker, the amino acid sequence of each of said polypeptide domains comprising a set of CDRs interposed between a set of FRs, each of which is respectively homologous with at least a portion of CDRs and FRs from an immunoglobulin molecule,
said polypeptide linker comprising plural, peptide-bonded amino acids defining a polypeptide of a length sufficient to span the distance between the C-terminal end of one of said domains and the N-terminal end of the other of said domains when said binding protein assumes a conformation suitable for binding, and comprising hydrophilic amino acids which together assume an unstructured polypeptide configuration in aqueous solution,
said binding protein being capable of binding to a preselected antigenic site, determined by the collective tertiary structure of said sets of CDRs held in proper conformation by said sets of FRs and said linker when disposed in aqueous solution.

## Claims

1. A single polypeptide chain comprising a linking sequence of a length of at least 10 amino acid residues, the linking sequence connecting a first and a second non-naturally peptide-bonded, biologically active polypeptide domain to form a single polypeptide chain comprising at least two biologically active domains connected by said linking sequence, said linking sequence comprising hydrophilic peptide-bonded amino acids exhibiting small and unreactive side chains but no cysteine, the hydrophilic amino acids constituting a hydrophilic sequence having a flexible unstructured configuration essentially free of secondary structure in aqueous solution, the linking sequence having a plurality of glycine or serine residues and spanning the distance between the C-terminal end of the first domain and the N-terminal end of the second domain.

2. The polypeptide chain of claim 1, wherein said linking sequence comprises threonine.

3. The polypeptide chain of claim 1 or 2, further comprising said first domain connected by a peptide bond to said N-terminal end of said linking sequence and a second domain connected by a peptide bond to the C-terminal end of said linking sequence.

4. The polypeptide chain of claim 1, wherein said linking sequence comprises plural consecutive copies of an amino acid sequence.

5. The polypeptide chain of claim 4, comprising the amino acid sequence (GlyGlyGlyGlySer)₃.

6. The polypeptide chain of claim 1, wherein said linking sequence comprises one or a pair of amino acid sequences recognizable by a site-specific cleavage agent.

7. DNA encoding the polypeptide chain of any of the preceding claims.

8. A polypeptide linker, the linker having a length of at least 10 amino acid residues and linking two non-naturally linked polypeptide domains to form a multifunctional protein, said linker exhibiting amino acids with small and unreactive side chains and comprising plural hydrophilic peptide-bonded amino acids constituting a hydrophilic sequence, said linker spanning the distance between the C-terminal end of a first domain and the N-terminal end of a second domain, wherein each said domain comprises a biologically active polypeptide having a conformation suitable for biological activity independent of the biological activity of the other domain.

9. A polypeptide linker, the linker having a length of at least 10 amino acid residues and linking two non-naturally linked polypeptide domains to form a functional protein, said linker exhibiting amino acids with small and unreactive side chains and comprising plural hydrophilic peptide-bonded amino acids constituting a hydrophilic sequence, said linker spanning the distance between the C-terminal end of a first domain and the N-terminal end of a second domain, wherein said domains together comprise an immunologically reactive binding site specific for a preselected antigen.

10. The polypeptide linker of claim 9, wherein said two domains mimic a v_{H} and v_{L} chain from a natural immunoglobulin.

11. The polypeptide linker of claim 8 or 9, which linker
(a) comprises threonine, or
(b) is cysteine-free, or
(c) comprises a plurality of glycine or serine residues, or
(d) comprises plural consecutive copies of an amino acid sequence, or
(e) spans a distance of at least 4 nm (40 Ångstroms), or
(f) comprises the amino acid sequence GlyGlyGlyGlySerGlyGlyGlyGlySerGlyGlyGly GlySer, or
(g) comprises one amino acid sequence or a pair of amino acid sequences recognizable by a site-specific cleavage agent.

12. The polypeptide linker of claim 8, wherein at least one of said domains comprises an enzyme, a toxin, a receptor, a binding site, a biosynthetic antibody binding site, a growth factor, a cell-differentiation factor, a lymphokine, a cytokine, a hormone, a remotely detectable moiety, or an anti-metabolite.

13. The polypeptide linker of claim 8, wherein said first domain comprises a single chain binding site and said second domain comprises an enzyme, a toxin, a receptor, a binding site, a biosynthetic antibody binding site, a growth factor, a cell-differentiation factor, a lymphokine, a cytokine, a hormone, or an anti-metabolite.

14. The polypeptide linker of claim 8, wherein at least one of said domains comprises a polypeptide capable of sequestering an ion.

15. The polypeptide linker of claim 14, wherein the polypeptide comprises calmodulin, methallothionein, a fragment thereof, or an amino acid sequence rich in at least one of glutamic acid, aspartic acid, lysine, and arginine.

16. The polypeptide linker of claim 8 or 9, wherein the amino acids of said linker together assume an unstructured polypeptide configuration in aqueous solution.

17. DNA encoding the polypeptide linker of claim 8 or 9.

18. Host cell transformed with and capable of expressing the DNA of claim 17.

## Patentansprüche

1. Eine einzelne Polypeptid-Kette, umfassend eine verbindende Sequenz mit einer Länge von wenigstens 10 Aminosäure-Resten, wobei die verbindende Sequenz eine erste und zweite nicht-natürliche peptid-gebundene biologisch aktive Polypeptid-Domäne unter Bildung einer einzelnen Polypeptid-Kette verbindet, wobei die einzelne Polypeptid-Kette wenigstens zwei biologisch aktive Domänen, verknüpft über die verbindende Sequenz, umfaßt, wobei die verbindende Sequenz hydrophile peptid-gebundene Aminosäuren mit kleinen und nicht-reaktiven Seitenketten, aber kein Cystein umfaßt, und wobei die hydrophilen Aminosäuren eine hydrophile Sequenz mit einer flexiblen unstrukturierten Konfiguration, die in wäßriger Lösung im Wesentlichen frei von Sekundärstrukturen ist, ausbilden, und wobei die verbindende Sequenz eine Vielzahl von Glyzin- oder Serin-Resten enthält und den Abstand zwischen dem C-terminalen Ende der ersten Domäne und dem N-terminalen Ende der zweiten Domäne überbrückt.

2. Die Polypeptid-Kette nach Anspruch 1, wobei die verbindende Sequenz Threonin umfaßt.

3. Die Polypeptid-Kette nach Anspruch 1 oder 2, weiterhin umfassend die erste Domäne, mittels Peptid-Bindung verknüpft mit dem N-terminalen Ende der verbindenden Sequenz, und eine zweite Domäne, verknüpft mittels Peptid-Bindung mit dem C-terminalen Ende der verbindenden Sequenz.

4. Die Polypeptid-Kette nach Anspruch 1, wobei die verbindende Sequenz eine Vielzahl aufeinanderfolgender Kopien einer Aminosäure-Sequenz umfaßt.

5. Die Polypeptid-Kette nach Anspruch 4, umfassend die Aminosäure-Sequenz (GlyGlyGlyGlySer)₃.

6. Die Polypeptid-Kette nach Anspruch 1, wobei die verbindende Sequenz eine oder ein Paar von Aminosäure-Sequenzen umfaßt, die von einem ortsspezifischen Spaltungsmittel erkannt wird/werden.

7. DNA, kodierend für die Polypeptid-Kette nach einem der vorhergehenden Ansprüche.

8. Ein Polypeptid-Linker mit einer Länge von wenigstens zehn Aminosäure-Resten, der zwei natürlicherweise nicht verbundene Polypeptid-Domänen unter Bildung eines multifunktionalen Proteins miteinander verbindet und Aminosäuren mit kleinen und nicht-reaktiven Seitenketten sowie mehrere hydrophile Peptid-gebundene Aminosäuren, die eine hydrophile Sequenz ausbilden, umfaßt, wobei der Linker den Abstand zwischen dem C-terminalen Ende einer ersten Domäne und dem N-terminalen Ende einer zweiten Domäne überbrückt, und wobei jede dieser beiden Domänen ein biologisch aktives Polypeptid mit einer für eine biologische Aktivität unabhängig von der biologischen Aktivität der anderen Domäne geeignete Konformation aufweist.

9. Ein Polypeptid-Linker mit einer Länge von wenigstens zehn Aminosäure-Resten, der zwei natürlicherweise nicht verbundene Polypeptid-Domänen unter Bildung eines funktionalen Proteins miteinander verbindet und Aminosäuren mit kleinen und nicht-reaktiven Seitenketten sowie mehrere hydrophile Peptid-gebundene Aminosäuren, die eine hydrophile Sequenz ausbilden, umfaßt, wobei der Linker den Abstand zwischen dem C-terminalen Ende einer ersten Domäne und dem N-terminalen Ende einer zweiten Domäne überbrückt, und wobei die Domänen gemeinsam eine immunologisch reaktive Bindungsstelle, spezifisch für ein bestimmtes Antigen, umfassen.

10. Der Polypeptid-Linker nach Anspruch 9, wobei die beiden Domänen eine V_{H}- bzw. eine V_{L}-Kette eines natürlichen Immunglobulins imitieren.

11. Der Polypeptid-Linker nach Anspruch 8 oder 9, wobei der Linker
(a) Threonin umfaßt, oder
(b) frei von Cystein ist, oder
(c) eine Vielzahl von Glycin-oder Serin-Resten umfaßt, oder
(d) mehrere aufeinander folgende Kopien einer Aminosäure-Sequenz umfaßt, oder
(e) einen Abstand von wenigstens 4 nm (40 Ångström) überbrückt, oder
(f) die Aminosäure-Sequenz GlyGlyGlyGlySerGlyGlyGlyGlySerGlyGlyGlyGlySer umfaßt, oder
(g) eine Aminosäure-Sequenz oder ein Paar von Aminosäure-Sequenzen, die von einem ortspezifischen Spaltungsmittel erkannt wird/werden, umfaßt.

12. Der Polypeptid-Linker nach Anspruch 8, wobei mindestens eine der beiden Domänen ein Enzym, ein Toxin, einen Rezeptor, eine Bindungsstelle, eine Bindungsstelle eines biosynthetischen Antikörpers, einen Wachstumsfaktor, einen Zelldifferenzierungsfaktor, ein Lymphokin, ein Cytokin, ein Hormon, eine indirekt nachweisbare Einheit oder einen Anti-Metaboliten umfaßt.

13. Der Polypeptid-Linker nach Anspruch 8, wobei die erste Domäne eine Einzelketten-Bindungsstelle und die zweite Domäne ein Enzym, ein Toxin, einen Rezeptor, eine Bindungsstelle, eine Bindungsstelle eines biosynthetischen Antikörpers, einen Wachstumsfaktor, einen Zelldifferenzierungsfaktor, ein Lymphokin, ein Cytokin, ein Hormon oder einen Anti-Metaboliten umfassen.

14. Der Polypeptid-Linker nach Anspruch 8, wobei mindestens eine der beiden Domänen ein Polypeptid umfaßt, das ein Ion maskieren kann.

15. Der Polypeptid-Linker nach Anspruch 14, wobei das Polypeptid Calmodulin, Methallothionein, ein Fragment davon oder eine Aminosäure-Sequenz umfaßt, die reich ist an zumindest einer der Aminosäuren Glutaminsäure, Asparaginsäure, Lysin und Arginin.

16. Der Polypeptid-Linker nach Anspruch 8 oder 9, wobei die Aminosäuren des Linkers in wäßriger Lösung gemeinsam eine unstrukturierte Polypeptid-Konfiguration annehmen.

17. DNA, kodierend für den Polypeptid-Linker nach Anspruch 8 oder 9.

18. Wirtszelle, transformiert mit und befähigt zur Expression der DNA nach Anspruch 17.

## Revendications

1. Une chaîne polypeptidique unique comprenant une séquence de liaison d'une longueur de 10 résidus d'acides aminés au moins, la séquence de liaison reliant un premier et un deuxième domaines polypeptidiques biologiquement actifs non liés de façon peptidique à l'état naturel, de manière à former une chaîne polypeptidique unique comprenant au moins deux domaines biologiquement actifs reliés par ladite séquence de liaison, ladite séquence de liaison comprenant des acides aminés hydrophiles liés de façon peptidique présentant des chaînes latérales courtes et non réactives mais aucune cystéine, les acides aminés hydrophiles constituant une séquence hydrophile présentant une configuration non structurée flexible essentiellement dépourvue de structures secondaires en solution aqueuse, la séquence de liaison ayant une pluralité de résidus glycine ou sérine et couvrant la distance entre l'extrémité C-terminale du premier domaine et l'extrémité N-terminale du second domaine.

2. La chaîne polypeptidique selon la revendication 1, **caractérisée en ce que** ladite séquence de liaison comprend de la thréonine.

3. La chaîne polypeptidique selon la revendication 1 ou 2, comprenant en outre ledit premier domaine lié par une liaison peptidique à ladite extrémité N-terminale de ladite séquence de liaison et un second domaine lié par une liaison pèptidique à l'extrémité C-terminale de ladite séquence de liaison.

4. La chaîne polypeptidique selon la revendication 1, **caractérisée en ce que** ladite séquence de liaison comprend plusieurs copies consécutives d'une séquence en acides aminés.

5. La chaîne polypeptidique selon la revendication 4, comprenant la séquence d'acides aminés (GlyGlyGlyGlySer)₃.

6. La chaîne polypeptidique selon la revendication 1, **caractérisée en ce que** ladite séquence de liaison comprend une séquence ou une paire de séquences d'acides aminés reconnaissable(s) par un agent de clivage site-spécifique.

7. ADN codant pour la chaîne polypeptidique selon l'une quelconque des revendications précédentes.

8. Un agent de couplage polypeptidique, l'agent ayant une longueur d'au moins 10 résidus d'acides aminés et reliant deux domaines polypeptidiques non naturellement liés, de manière à former une protéine multi-fonctionnelle, ledit agent présentant des acides aminés avec des chaînes latérales courtes et non réactives et comprenant plusieurs acides aminés hydrophiles liés de façon peptidique constituant une séquence hydrophile, ledit agent couvrant la distance entre l'extrémité C-terminale d'un premier domaine et l'extrémité N-terminale d'un second domaine, chacun desdits domaines comprenant un polypeptide actif sur le plan biologique présentant une conformation adaptée pour une activité biologique indépendante de l'activité biologique de l'autre domaine.

9. Un agent de couplage polypeptidique, l'agent ayant une longueur d'au moins 10 résidus d'acides aminés et reliant deux domaines polypeptidiques non naturellement liés, de manière à former une protéine fonctionnelle, ledit agent présentant des acides aminés avec des chaînes latérales courtes et non réactives et comprenant plusieurs acides aminés hydrophiles liés de façon peptidique constituant une séquence hydrophile, ledit agent couvrant la distance entre l'extrémité C-terminale d'un premier domaine et l'extrémité N-terminale d'un second domaine, lesdits domaines comprenant ensemble un site de liaison réactif sur le plan immunologique et spécifique pour un antigène pré-sélectionné.

10. L'agent polypeptidique selon la revendication 9, **caractérisé en ce que** lesdits deux domaines miment une chaîne V_{H} et V_{L} d'une immunoglobuline naturelle.

11. L'agent polypeptidique selon la revendication 8 ou 9, ledit agent
(a) comprend de la threonine, ou
(b) est dépourvu de cystéine, ou
(c) comprend plusieurs résidus glycine ou sérine, ou
(d) comprend plusieurs copies consécutives d'une séquence d'acides aminés, ou
(e) couvre une distance d'au moins 4 nm (40 Ångstroms), ou
(f) comprend la séquences d'acides aminés GlyGlyGlyGlySerGlyGlyGlyGlySerGlyGlyGlyGlySer, ou
(g) comprend une séquence d'acides aminés ou une paire de séquences d'acides aminés reconnaissable(s) par un agent de clivage site-spécifique.

12. L'agent polypeptidique selon la revendication 8, **caractérisé en ce qu'**au moins l'un desdits domaines comprend, un enzyme, une toxine, un récepteur, un site de liaison, un site de liaison à un anticorps biosynthétique, un facteur de croissance, un facteur de différentiation cellulaire, une lymphokine, une cytokine, une hormone, une portion détectable amovible, ou un anti-métabolite.

13. La liaison polypeptidique selon la revendication 8, **caractérisée en ce que** ledit premier domaine comprend un site de liaison à une chaîne unique et ledit second domaine comprend un enzyme, une toxine, un récepteur, un site de liaison, un site de liaison biosynthétique d'anticorps, un facteur de croissance, un facteur de différentiation cellulaire, une lymphokine, une cytokine, une hormone, ou un anti-métabolite.

14. L'agent polypeptidique selon la revendication 8, **caractérisé en ce qu'**au moins l'un desdits domaines comprend un polypeptide capable de séquestrer un ion.

15. L'agent polypeptidique selon la revendication 14, **caractérisé en ce que** le polypeptide comprend la calmoduline, la methallothionéine, un fragment de celles-ci, ou une séquence d'acides aminés riche en l'un au moins des acides aminés acide glutamique, acide aspartique, lysine et arginine.

16. L'agent polypeptidique selon la revendication 8 ou 9, **caractérisé en ce que** les acides aminés dudit agent prennent ensemble une configuration polypeptidique non structurée en solution aqueuse.

17. ADN codant pour l'agent polypeptidique selon la revendication 8 ou 9.

18. Cellule hôte transformée avec et capable d'exprimer l'ADN selon la revendication 17.
